# EUROPEAN PATENT APPLICATION

(11) **EP 2 330 218 A1**
(43) Date of publication of application: **08.06.2011**
(21) Application number: 09382271.6
(22) Date of filing: 07.12.2009
(51) Int. Cl.: C12Q 1/68

(54) **EGFR and PTEN gene alterations predicts survival in patients with brain tumors**

(71) Applicant: Europath Biosciences, S.L., 08025 Barcelona (ES)
(72) Inventor: Donovan, Michael J., 08025 Barcelona (ES); Colomer Valero, Anna, 08025 Barcelona (ES); Erill Sagalés, Nadina, 08025 Barcelona (ES); Ferrer Abizanda, Isidre, 08907 Hospitalet de Llobregat Barcelona (ES); Boluda Casas, Susana, 08907 Hospitalet de Llobregat Barcelona (ES)
(74) Representative: ABG Patentes, S.L.

(57) **Abstract**

The invention relates to methods of predicting the clinical outcome of brain cancer patients based on the expression levels and/or the LOH levels of PTEN gene and on the expression levels and/or the polysomy / amplification levels of EGFR gene in a sample from said patients.

## Description

### FIELD OF THE INVENTION

The invention relates to the fields of diagnostics and therapeutics, in particular to a method of providing personalized management to brain cancer patients based on the expression of certain genes in a sample from said patients, which certain serve as treatment targets.

### BACKGROUND OF THE INVENTION

### Gliomas: Diagnosis and disease categorization.

A glioma is a type of cancer that starts in the brain or spine. It is called a glioma because it arises from glial cells and/or its precursors. The most common site of gliomas is the brain. Gliomas are classified by cell type, by grade, and by location. Gliomas are named according to the specific type of cell they most closely resemble. The main types of gliomas are:
- Ependymomas, gliomas derived from ependymal cells
- Astrocytomas, gliomas derived from astrocytes. The glioblastoma multiforme is the most common astrocytoma.
- Oligodendrogliomas , gliomas derived from oligodendrocytes.
- Mixed gliomas, such as oligoastrocytomas, that contain cells from different types of glia.

Gliomas are further categorized according to their grade, which is determined by pathologic evaluation of the tumor. Thus we can distinguish between low-grade gliomas that are well-differentiated (not anaplastic), are benign and portend a better prognosis for the patient; and high-grade gliomas, that are undifferentiated or anaplastic, are malignant and carry a worse prognosis.

Of numerous grading systems in use, the most common is the World Health Organization (WHO) grading system for astrocytoma.

### Treatment of brain gliomas.

The treatment for brain gliomas depends on the location, the cell type and the grade of malignancy. Often, treatment is a combined approach, using surgery, radiation therapy, and chemotherapy. The radiation therapy is in the form of external beam radiation or the stereotactic approach using radiosurgery. Spinal cord tumors can be treated by surgery and radiation. Temozolomide is a chemotherapeutic drug that is able to cross the blood-brain barrier effectively and is being used in therapy. Despite these approaches most high grade glioma patients succumb to their disease. New therapeutic interventions to critical targets are needed to improve outcome in this patient population.

### Glioblastoma multiforme

The glioblastoma multiforme (GBM, WHO grade IV) is a highly aggressive brain tumor presenting as one of two subtypes with distinct clinical histories and molecular profiles. The primary GBM presents acutely as a high-grade disease and the secondary GBM subtype evolves from the slow progression of a low-grade disease.

Brown et al (J. of clinical Oncology., 2008, 5603-5609) describe a phase I/II trial of erlotinib combined with temozolomide in patients suffering glioblastoma multiforme (GBM). These authors tried to correlate the response of the patients with several molecular markers like EGFR, PTEN, P53 etc. but failed to observe any correlation between survival and expression levels of said genes.

Mirimanoff R.O et al (J Clin Oncology 2006. 2563-2569) describe a completed EORTC PhIII trial, where the MGMT promoter methylation was the strongest predictor for outcome and positive response to temozolomide.

Van den Bent M. J. et al (JCO 2009;27:1268-1274) describes a recent randomized PHII EORTC trial. Patients with progressive GBM after prior radiotherapy were randomly assigned to either erlotinib or a control arm that received treatment with either temozolomide or carmustine (BCNU). The primary end point was 6-month progression-free survival (PFS). Tumor specimens obtained at first surgery were investigated for EGFR expression; EGFRvIII mutants; EGFR amplification; EGFR mutations in exons 18, 19, and 21; and pAkt. No clear biomarker associated with improved outcome to erlotinib was identified.

Smith J.S et al (J. National Cancer Institute 2001. 1246-1256) describe methods for predicting the survival of patients with anaplastic astrocytoma and glioblastoma multiforme. These authors identify that mutation of PTEN and EGFR amplification are independent prognostic markers for patients with anaplastic astrocytoma and EGFR amplification is a survival marker for older patients with gliobastoma multiforme.

Prados M. D. et al (J Clin Oncol. 2009 27(4):579-84) describes a Ph II trial which evaluated erlotinib plus temozolomide during and after radiation, patients treated with combination therapy (i.e. erlotinib and temozolomide) had better survival. In addition, the study also evaluated several biomarkers and found that methylation of the MGMT promoter along with PTEN expression was associated with improved survival.

However, there is still a need for further markers and markers combinations useful for predicting the clinical outcome of the glioma patients. A special area for diagnosis and prognosis is the study of the biopsy sample. An integrated approach able to better define patient outcome based on the most appropriate treatment using tumor profiles will be critical, offering in addition to the glioma patient a better quality of life.

### SUMMARY OF THE INVENTION

In a first aspect, the invention relates to a method for predicting the clinical outcome of a subject suffering from glioma comprising determining the expression levels of EGFR and/or polysomy and/or amplification of the EGFR locus on chromosome 7 and the expression levels and/or the LOH levels of PTEN gene in a sample from the subject and comparing said expression levels or the polysomy / amplification levels of EGFR gene and the expression levels and/or the LOH levels of PTEN gene with standard reference values, wherein low expression levels and/or high LOH levels of PTEN gene with respect to said reference values and high expression levels and/or high polysomy / amplification of EGFR gene are indicative of a good clinical outcome of the subject.

In a second aspect, the invention relates to a method for predicting the clinical outcome of a subject suffering from glioma comprising determining the expression levels and/or the LOH levels of PTEN gene in a sample from the subject and comparing said expression levels and/or the LOH levels of PTEN gene with standard reference values, wherein low expression levels and/or low LOH levels of PTEN gene with respect to said reference values, is indicative of a poor clinical outcome of the subject.

In a third aspect, the invention relates to a Kit comprising agents capable of specifically detecting the expression levels and/or the polysomy of EGFR and the expression levels and/or the LOH of PTEN genes.

### BRIEF DESCRIPTION OF THE FIGURES

Figure 1. Kaplan-Meier survival curve illustrating time from first surgery to death / end-of follow-up of only (Pure) GBM patients, astrocyoma patients and oligodendroglioma patients.
Figure 2. Kaplan-Meier curve estimating survival from first surgery to death / recurrence / progression or end-of-follow-up of only (Pure) GBM patients, astrocyoma patients and oligodendroglioma patients.
Figure 3. Kaplan-Meier survival curve demonstrating stratification of patients in the glioblastoma (GBM) /anaplastic astrocytoma and mixed group with PTEN LOH (p=0.04).
Figure 4. Kaplan-Meier survival curve demonstrating stratification of patients in the glioblastoma (GBM) /anaplastic astrocytoma and mixed group as having both EGFR AMP/HP (amplification - high polysomy) and PTEN LOH (p=0.034).
Figure 5. Representative images of glioblastoma multiforme specimens stained with H&E (Hematoxylin and Eosin) (A). Example of FISH experiments where AMP/HP EGFR DNA FISH (B) and monosomy PTEN (C) are illustrated.

### DETAILED DESCRIPTION OF THE INVENTION

In order to facilitate the understanding of the invention described in this patent application, the meaning of some terms and expressions in the context of the invention are explained below:
The term "subject" refers to a member of a mammal animal species, and includes, but is not limited thereto, domestic animals, primates and humans; the subject is preferably a human being, male or female, of any age or race. Alternatively, the term "individual" is also sometimes used in this description to refer to human beings.
The term "protein" refers to a molecular chain of amino acids, linked by covalent or non-covalent bonds. The term includes all the forms of post-translational modifications, for example, glycosylation, phosphorylation or acetylation.
The term "antibody" refers to a protein with the capacity to specifically bind to an "antigen". The term "antibody" comprises recombinant antibodies, monoclonal antibodies, or polyclonal antibodies, intact, or fragments thereof which maintain the capacity to bind to the antigen, combibodies, etc., both human or humanised and of non-human origin.
The term "primer oligonucleotide", as used in the present invention, refers to a nucleotide sequence, which is complementary to a nucleotide sequence of a selected gene. Each primer oligonucleotide hybridises with its target nucleotide sequence and acts as an initiation point for DNA polymerisation.

The authors of the present invention have found that the clinical outcome of patients suffering from glioma cancer correlates with levels and/or the polysomy / amplification levels of EGFR gene and the expression levels and/or the LOH levels of PTEN.

Thus, in a first aspect, the invention relates to a method for predicting the clinical outcome of a subject suffering from glioma that comprises
a) determining the expression levels and/or the polysomy/ amplification levels of EGFR gene and the expression levels and/or the LOH levels of PTEN gene in a sample from the subject and
b) comparing said expression levels or the polysomy / amplification levels of EGFR gene and the expression levels and/or the LOH levels of PTEN gene with standard reference values,
wherein low expression levels and/or high LOH levels of PTEN gene with respect to said standard reference values and high expression levels and/or high polysomy of EGFR gene with respect to said standard reference values are indicative of a good clinical outcome of the subject. In the present invention a "glioma" is a type of cancer that starts in the brain or spine. It is called a glioma because it arises from glial cells. The most common site of gliomas is the brain. Gliomas are classified by cell type, by grade, and by location.

Gliomas are named according to the specific type of cell they most closely resemble. The main types of gliomas are:
- Ependymomas, gliomas derived from ependymal cells
- Astrocytomas, gliomas derived from astrocytes. Astrocytoma are derived in pilocytic, diffuse, anaplastic, and glioblastoma multiforme. The glioblastoma multiforme is the most common astrocytoma.
- Oligodendrogliomas , gliomas derived from oligodendrocytes.
- Mixed gliomas, such as oligoastrocytomas, that contain cells from different types of glia.

In a preferred embodiment, the glioma is a gliobastoma multiforme (GBM) and more preferably the gliobastoma is an early gliobastoma.

The gliobastoma multiforme is the most common and malignant form of glial tumors and is composed of a heterogenous mixture of poorly differentiated malignant astrocytes and dysplastic endothelial cells. It primarily effects adults, involves the cerebral hemispheres and has a rapid disease course which often leads to death.

In the present invention "clinical outcome" is understood as the expected course of a disease. It denotes the doctor's prediction of how a subject's disease will progress, and whether there is chance of recovery or recurrence.

The prediction of the clinical outcome can be done by using any endpoint measurements used in oncology and known to the skilled practitioner. Useful endpoint parameters to describe the evolution of a disease include:
- disease-free progression which, as used herein, describes the proportion of patients in complete remission who have had no recurrence of disease during the time period under study.
- objective response, which, as used in the present invention, describes the proportion of treated people in whom a complete or partial response is observed.
- tumor control, which, as used in the present invention, relates to the proportion of treated people in whom complete response, partial response, minor response or stable disease ≥ months is observed.
- progression free survival which, as used herein, is defined as the time from start of treatment to the first measurement of cancer growth.
- six-month progression free survival or PFS6" rate which, as used herein, relates to the percentage of people wherein free of progression in the first six months after the initiation of the therapy and
- median survival which, as used herein, relates to the time at which half of the patients enrolled in the study are still alive.

A good clinical outcome is understood as a situation where at least 10%, 20%, 30%, 40%, 50%, 60%, 70%, 80%, 90% of the patients have a positive result regarding the endpoint parameters described above.

The term "sample" as used herein, relates to any sample which can be obtained from the patient. The present method can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In a particular embodiment, said sample is a tumour tissue sample or portion thereof In a more particular embodiment, said tumor tissue sample is a brain tumor tissue sample from a patient suffering from brain cancer. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The samples may be obtained from subjects previously diagnosed with glioma (patients), or from subjects who have not been previously diagnosed with glioma, or from patients diagnosed with glioma who are undergoing treatment, or from subjects diagnosed with glioma who have been previously treated.

In a preferred embodiment, the sample is a tumor sample that contains a substantially number of tumor cells.

PTEN is the Phosphatase and tensin homolog protein also known as BZS; MHAM; TEP1; MMAC1; PTEN1; 10q23del or MGC11227. The PTEN is a protein (which in humans is encoded by the PTEN gene (RefSEq ID NM_000314 SEQ ID NO: 1, Protein reference NP_000305.3 SEQ ID NO: 2) (Steck PA, et al. 1997 Nat. Genet. 15 (4): 356-62 2). PTEN acts as a tumor suppressor gene through the action of its phosphatase protein product. This phosphatase is involved in the regulation of the cell cycle, preventing cells from growing and dividing too rapidly. Mutations of this gene contribute to the development of certain cancers (Chu EC, et al. 2004 Med. Sci. Monit. 10 (10): RA235-41 3). It does exist homologs in other species (mice (NM_008960.2, SEQ ID NO: 3), rat (NM 031606.1, SEQ ID NO: 4) and dog (NM 001003192, SEQ ID NO: 5), etc).

The protein encoded this gene is a phosphatidylinositol-3,4,5-trisphosphate 3-phosphatase. It contains a tensin like domain as well as a catalytic domain similar to that of the dual specificity protein tyrosine phosphatases. Unlike most of the protein tyrosine phosphatases, this protein preferentially dephosphorylates phosphoinositide substrates. It negatively regulates intracellular levels of phosphatidylinositol-3,4,5-trisphosphate in cells and functions as a tumor suppressor by negatively regulating Akt/PKB signaling pathway (Hamada K, et al 2005 Genes Dev 19 (17): 2054-65)

The epidermal growth factor receptor (EGFR; ErbB-1; HER1 in humans) is the cell-surface receptor for members of the epidermal growth factor family (EGF-family) of extracellular protein ligands. (Herbst RS (2004). Int. J. Radiat. Oncol. Biol. Phys. 59 (2 Suppl): 21-6. 1) The epidermal growth factor receptor is a member of the ErbB family of receptors. The EGFR is a protein which in humans is encoded by different isoforms: EGFR transcript variant 1(NM_005228.3, SEQ ID NO: 6), transcript variant 2 (NM_201282.1, SEQ ID NO: 7), transcript variant 3 (NM 201283.1, SEQ ID NO: 8) and transcript variant 4 (NM_201284.1, SEQ ID NO: 9). It does exist homologes in other species (mice (NM_207655.2, SEQ ID NO: 10 and NM 007912.4, SEQ ID NO: 11), rat (NM_031507.1, SEQ ID NO: 12) and dog (XM_533073.2, SEQ ID NO: 13), etc)

The method of the invention requires determining the expression levels of the EGFR gene and PTEN gene. As the person skilled in the art understands, the expression levels of the PTEN gene, and EGFR gene can be determined by measuring the levels of mRNA encoded by said genes, measuring both the levels of proteins encoded by said genes, i.e. EGFR protein, and PTEN protein, and the levels of variants thereof and through the use of surrogates (DNA copy number) for associating gene level with mRNA and protein product of said gene.

Variants of the EGFR protein, and PTEN protein may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the protein is an alternative splice variant of the proteins of the present invention and/or (iv) fragments of the proteins. The fragments include proteins generated via proteolytic cleavage (including multi-site proteolysis) of an original sequence. Variants are deemed to be within the scope of those skilled in the art from the teaching herein.

As known in the art the "similarity" between two proteins is determined by comparing the amino acid sequence and its conserved amino acid substitutes of one protein to a sequence of a second protein. Variants according to the present invention includes amino acid sequences that are at least 60%, 65%, 70%, 72%, 74%, 76%, 78%, 80%, 90%, or 95% similar or identical to the original amino acid sequence. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

In a preferred embodiment, the determination of the expression levels of the EGFR gene and PTEN gene can be carried out by measuring the expression levels of the mRNA encoded by the EGFR gene and PTEN gene. For this purpose, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

In a particular embodiment, the expression level is determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing aspects of the invention, the gene mRNA expression levels are often determined by reverse transcription polymerase chain reaction (RT-PCR). The detection can be carried out in individual samples or in tissue micro arrays.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA are mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin. In a preferred embodiment, the control RNA is β-actin mRNA. In one embodiment relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene.

The determination of the level of expression of the EGFR gene and PTEN gene needs to be correlated with the standard reference values. Standard reference values which correspond to the median value of expression levels of EGFR gene and PTEN gene measured in a collection of samples from healthy patients. Once this median value is established, the level of this marker expressed in tumor tissues from patients can be compared with this median value, and thus be assigned a level of "low," "normal" or "high". The collection of samples from which the reference level is derived will preferably be constituted from healthy persons from the same age as the patients. In any case it can contain a different number of samples. In a more preferred embodiment, the samples are biopsy brain samples. Preferably the collection should be sufficient to provide an accurate reference level. Preferably the number of samples used for make the reference values is more than 10, 20, 30, 40, 50, 60, 70, 80, 90, 100, 150, 200, 500 samples.

In a particular embodiment, an increase in expression above the reference value of at least 1.1-folk, 1.5-fold, 5-fold, 10-fold, 20-fold, 30-fold, 40-fold, 50-fold, 60-fold, 70-fold, 80-fold, 90-fold, 100-fold or even more compared with the reference value is considered as "high" expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "low" expression.

Alternatively, in another particular embodiment, the expression levels of the EGFR gene, and/or PTEN gene can be determined by measuring both the levels of proteins encoded by said genes, i.e. EGFR protein, and PTEN protein, and the levels of variants thereof.

The determination of the expression levels of the PTEN or EGFR proteins and the variants thereof can be carried out by immunological techniques such as e.g. ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression and subcellular localization by microscopy. Generally, the cells under study are previously fixed with paraformaldehyde and permeabilised with a non-ionic detergent. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry. This technique does not allow the exact localisation of the target protein or the determination of its molecular weight but allows a very specific and highly sensitive detection of the target protein in a variety of biological samples (serum, plasma, tissue homogenates, postnuclear supernatants, ascites and the like). In a preferred embodiment, the EGFR and PTEN proteins are detected by immunohistochemistry (IHC) analysis using thin sections of the biological sample immobilised on coated slides. The sections are then deparaffinised, if derived from a paraffinised tissue sample, and treated so as to retrieve the antigen. The detection can be carried out in individual samples or in tissue microarrays. In another embodiment the method used is a proteomic array

Any antibody or reagent known to bind with high affinity to the target protein can be used for detecting the amount of target protein. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

In yet another embodiment, the determination of EGFR and PTEN proteins expression levels can be carried out by constructing a tissue microarray (TMA) containing the patient samples assembled, and determining the expression levels of EGFR and PTEN proteins by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cutoffs were selected in order to facilitate reproducibility, and when possible, to translate biological events.

The determination of the level of expression of the EGFR gene and PTEN gene needs to be correlated with the reference values which correspond to the median value of expression levels of EGFR gene and PTEN gene measured in a collection of brain tissue samples from healthy patients. Preferably the collection should be sufficient to provide an accurate reference level. Preferably the number of samples used for make the reference values is more than 10, 20, 30, 40, 50, 60, 70, 80, 90, 100, 150, 200, 500 samples.

In a preferred embodiment the sample is a biopsy. Once this median value is established, the level of this marker expressed in tumor tissues from patients can be compared with this median value, and thus be assigned a level of "low," "normal" or "high."

Polysomy and/or amplification of the EGFR locus on chromosome 7 in the present invention is understood as the presence of more than one copy of the EGFR locus on the chromosome 7.

In the present invention the term "LOH" refers to loss of heterozygosity. LOH in a cell represents the loss of normal function of one allele of a gene in which the other allele was already inactivated. This term is mostly used in the context of oncogenesis; after an inactivating mutation in one allele of a tumor suppressor gene occurs in the parent's germline cell, it is passed on to the zygote resulting in an offspring that is heterozygous for that allele. In oncology, loss of heterozygosity occurs when the remaining functional allele in a somatic cell of the offspring becomes inactivated by mutation. In the case of PTEN LOH, this results in no normal tumor suppressor being produced.

In a particular embodiment, the determination of the polysomy / amplification of EGFR and the LOH levels of PTEN can be measured in the DNA obtained from the tumor cells according to standard procedures such as quantitative PCR or comparative genomic hybridization to microarray technologies; or in the tumor cells from the paraffined-embedded section or from the cytology preparation by FISH using appropriate molecular probes. In a particular embodiment, the detection of the polysomy / amplification of EGFR and the LOH levels of PTEN is carried out by a hybridization-based assay. In a particular embodiment, the detecting step of the method of the invention comprises contacting the nucleic acid sample with one or more nucleic acid probes each of which selectively binds to a target polynucleotide sequence on the chromosome region of EGFR or PTEN, under conditions in which the probe forms a stable hybridization complex with the target polynucleotide sequence; and detecting the hybridization complex. In a particular embodiment, the nucleic acid probes used in the method of the present invention are labelled with a fluorophore.

In a particular embodiment, the step of detecting the hybridization complex comprises determining the copy number of the target polynucleotide sequence, thereby determining the presence of the polysomy or the LOH.

In a preferred embodiment of the invention, said hybridization-based assay is selected from the group consisting of Southern blot, PCR, in situ hybridization (ISH) fluorescence ISH (FISH) and comparative genomic hybridization (CGH). In a more preferred embodiment, the method is a comparative genomic hybridization assay.

In a particular embodiment of the invention, said hybridization-based assay is an array-based assay. In a particular embodiment, once the sample has been obtained and the total DNA has been extracted, genome-wide analysis of DNA copy number changes by comparative genomic hybridization (CGH) is carried out. In general, for a typical CGH measurement, total genomic DNA is isolated from test and reference cell populations, differentially labeled and hybridized to a representation of the genome that allows the binding of sequences at different genomic locations to be distinguished. Hybridization reactions can be performed under conditions of different stringency. The stringency of a hybridization reaction includes the difficulty with which any two nucleic acid molecules will hybridize to one another. Preferably, each hybridizing polynucleotide hybridizes to its corresponding polynucleotide under reduced stringency conditions, more preferably stringent conditions, and most preferably highly stringent conditions.

The amount of specimen DNA is frequently a constraint on CGH measurements. Typical array CGH procedures use 300 ng to 3 µg of specimen DNA in the labelling reaction, equivalent to approximately 50.000 to 500.000 mammalian cells. Usually, random primer labeling protocols are employed, which also amplifies the DNA, so that several micrograms are used in the hybridization.

Array CGH has been implemented using a wide variety of techniques. In a particular embodiment, array CGH is carried out using arrays from large-insert genomic clones such as bacterial artificial chromosomes (BACs). The general principles and conditions for detection of nucleic acids, such as using array CGH (comparative genomic hybridization (CGH) to BAC microarrays), are well known for the skilled person in the art. This technique allows scanning the entire genome for DNA copy number changes therefore allowing quantitative detection of DNA copy number variation in tumor genomes with high resolution (Pinkel D, et al. Nat Genet 1998; 20(2):207-11 and Hodgson G, et al. Nat Genet 2001;29(4):459-64).

As an illustrative non limitative example, in the array CGH carried out by the method of the present invention test tumor and reference genomic DNAs can be labeled by random priming using Cy3 and Cy5 fluorophores. Then, the images of the arrays may be analysed using, for example, a cooled coupled device (CCD) camera and appropriate software.

The major technical challenge of array CGH is generating hybridization signals that are sufficiently intense and specific so that copy number changes can be detected. The signal intensity on an array element is affected by a number of factors including the base composition, the proportion of repetitive sequence content, and the amount of DNA in the array element available for hybridization.

Array elements made from genomic BAC clones typically provide more intense signals than elements employing shorter sequences such as cDNAs, PCR products, and oligonucleotides. The higher signals from the more complex array elements result in better measurement precision, allowing detection of single-copy transition boundaries-even in specimens with a high proportion of normal cells.

In another preferred embodiment of the invention the method used is FISH (fluorescence in situ hybridization) or FISH plus spectral karotype (SKY) (Liehr T. et al 2008 Fluorescence In Situ Hybridization (FISH) - Application Guide, Springer Berlin Heidelberg)

FISH is a cytogenetic technique used to detect and localize the presence or absence of specific DNA sequences on chromosomes. FISH uses fluorescent probes that bind to only those parts of the chromosome with which they show a high degree of sequence similarity. A s **"probe"** is understood any ribopolynucleotide or desoxiribopolynucleotide sequence that specifically binds to only those parts of the chromosome with which they show a high degree of sequence similarity. The probe must be large enough to hybridize specifically with its target but not so large as to impede the hybridization process.

It does exist many different FISH probes that can be used in the present invention like without limitation, bacterial artificial chromosomes (BACs), Tiling Oligonucleotide Probes (TOPs), etc. The design of FISH probes is well know for a person skilled in the art (Bayani J, Squire JA..Curr Protoc Cell Biol. 2004 Sep; Chapter 22:Unit 22.4; Bayani J, Squire J.Curr Protoc Cell Biol. 2004 Oct;Chapter 22:Unit 22.5.; Navin, N. et al. Bioinformatics, Volume 22, Number 19, 1 October 2006 , pp. 2437-2438(2)) Publisher: Oxford University Press)

The probe can be tagged directly with fluorophores, with targets for antibodies or with biotin. Tagging can be done in various ways, such as nick translation, or PCR using tagged nucleotides.

The sample can be fixed and in paraffin embedded, thus an additionally step of deparafination may be performed.

For hybridization, an interphase or metaphase chromosome preparation is produced. The chromosomes are firmly attached to a substrate, usually glass. Repetitive DNA sequences must be blocked by adding short fragments of DNA to the sample. The probe is then applied to the chromosome DNA and incubated for approximately 12 hours while hybridizing. Several wash steps remove all unhybridized or partially-hybridized probes. After standard post hybridization washes the slides are stained with the DNA staining probe such DAPI and mounted with a mounting agent such as antifade.

The results are then visualized and quantified using a microscope that is capable of exciting the dye and recording images. If the fluorescent signal is weak, amplification of the signal may be necessary in order to exceed the detection threshold of the microscope. Fluorescent signal strength depends on many factors such as probe labeling efficiency, the type of probe, and the type of dye. Fluorescently-tagged antibodies or streptavidin are bound to the dye molecule. These secondary components are selected so that they have a strong signal. In a preferred embodiment, prior to imaging all slides are evaluated by a pathologist and regions of interest are identified based on histopathologic and quality criteria including, without excluding others, tumor content, appropriate fixation, necrosis and vascularity.

FISH experiments designed to detect or localize gene expression within cells and tissues rely on the use of a reporter gene, such as one expressing green fluorescent protein, to provide the fluorescence signal.

In an alternative technique to interphase or metaphase preparations, fiber FISH, interphase chromosomes are attached to a slide in such a way that they are stretched out in a straight line, rather than being tightly coiled, as in conventional FISH, or adopting a random conformation, as in interphase FISH. This is accomplished by applying mechanical shear along the length of the slide, either to cells that have been fixed to the slide and then lysed, or to a solution of purified DNA. The extended conformation of the chromosomes allows dramatically higher resolution - even down to a few kilobases.

In a preferred embodiment, parallel to the detection of the polysomy / amplification of EGFR locus and LOH of PTEN, FISH control probes for chromosomes 10 and 7 are used. Those "FISH control probes" are probes that binds specific for the individual chromosomes, thus allowing the determination of the chromosome number. In a preferred embodiment, the control probes are directed to alpha satellite sequences. Alpha satellite sequences, whilst highly repetitive, are specific to each individual chromosome. These sequences flank the centromeres and can present a target measured in megabases. In a preferred embodiment, these FISH control probes would be label with different colors than the EGFR and PTEN probes.

In a preferred embodiment, the PTEN FISH probe is a probe that hybridizes to the 10q23 region on chromosome 10 and contains sequences that flank both the 5' and 3' ends of the PTEN gene; in a more preferred embodiment the probe has between 300 and 400 kb. In a more preferred embodiment, the FISH probe for PTEN and the control probe for chromosome 10 are the LSI PTEN (10q23) / CEP 10 dual color probe for PTEN (Vysis, Abbot Molecular) (Goberdhan,D., et al.Human Molecular Genetics 12 (2) (2003): 239-248. Eng, C., et al.Human Mutation 22 (2003): 183-198. Sasaki, H., et al. Am. J. of Pathology 159 (1) (2001): 359-367). Other probes are described in Cairns et al. (1997. Cancer Res 57; 4997-5000) and Hermans et al, (2004 Genes Chrom Cancer, 39; 171-184). The LSIS PTEN (10q23) is labeled with SpectrumOrange. The CEP 10 SpectrumGreen probe hybridizes to alpha satellite sequences specific to chromosome 10.

The EGFR FISH probe is a probe that hybridizes to the 7p12 region of the chromosome 7 and that contains the entire EGFR gene. In a more preferred embodiment, the FISH probe for PTEN and the control probe for chromosome 10 are the LSI EGFR/CEP 7 dual color probe (Vysis, Abbot Molecular)

In a particular embodiment the EGFR Probe is labeled in SpectrumOrange and covers an approximately 300 kb region of the 7p12 (Bredel, M., et al. (1999) Clin Cancer Res 5, 1786-92. Harris, A., et al. (1989) J Steroid Biochem 34, 123-31. Kitagawa, Y., et al. (1996) Clin Cancer Res 2, 909-14.Neal, D.E., et al. (1990) Cancer 65, 1619-25. Osaki, A., et al. (1992) Am J of Surg 164, 323-6. Pavelic, K., et al. (1993) Anticancer Res 13, 1133-7. Sauter, G., et al. (1996) Am J Pathol 148, 1047-53. Torregrosa, D., et al. (1997) Clin Chim Acta 262, 99-119). The CEP 7 probe, labeled in SpectrumGreen, hybridizes to the alpha satellite DNA located at the centromere of chromosome 7 (7p11.1-q11.1).

Other methods may be used to determine copy number aberrations as are known in the art, including, but not limited to oligonucleotide-based microarrays (Lucito, et al. (2003) Genome Res. 13:2291-2305; Bignell et al. (2004) Genome Res. 14:287-295; Zhao, et al (2004) Cancer Research, 64(9):3060-71.

In another embodiment, the LOH is measured using simple sequence length polymorphisms (or microsatelites) (Virmani A.K. et al Genes chromosomes Cancer 1998, 21 (4) 308-319 or SNPS as genetic markers (Lindblad-toh K et al. Nat. Biothechnol 2000, 18(9)1001-1005).

The determination of the level of the polysomy / amplification of EGFR and the level of LOH of PTEN, needs to be correlated with a reference value. Said reference values are generated by the person skill in the art in form of a table that divide the patient in ascending number of copies of the EGFR gene or regarding to the LOH degree and ascending number of copies of the TEN gene.

For EGFR, the reference values can be generated, without limitation, using for example, the classification of Cappuzzo et al. (JNCI 2005;4:643-55). In this classification, the patients are classified into six strata with ascending number of copies of the EGFR gene per cell according to the frequency of tumor cells with specific number of copies of the EGFR gene per chromosome 7. As it was mentioned before, control probes that detect the chromosome 7 for obtaining a ratio number of copies of EGFRgene/chromosome 7 are commercially available. For example, the CEP 7 SpectrumGreen probe (Vysis) that hybridizes to alpha satellite sequences specific to chromosome 7, can be used.

An non limitative example of a table for classifying a patient attending to the polysomy of EGFR is:
1) disomy (D): ≤ copies in 90% of the cells of the sample;
2) low trisomy (LT): ≤ 2 copies in ≥ 40% of the cells of the sample or 3 copies in 10% - 40% of the cells of the sample or ≥ 4 copies in <10% of the cells of the sample;
3) high trisomy (HT): ≤ 2 copies in ≥ 40% of the cells of the sample or 3 copies in 40% of the cells of the sample or ≥ 4 copies in <10% of the cells of the sample;
4) low polysomy (LP): ≥ 4 copies in 10% - 40% of the cells of the sample;
5) high polysomy (HP): ≥ 4 copies in ≥ 40% of the cells of the sample or the presence of amplification (presence of tight EGFR gene clusters and a ratio of EGFR gene to chromosome of ≥ 2 or ≥ 15 copies of EGFR per cell in ≥ 10% of analyzed cells of the sample).

As a person skilled in the art would understand, the method of the invention can be made using more than one sample of a patient. In such a case, it is consider that exist "high polysomy levels of EGFR" when more than 50%, more than 60%, more than 70%, more than 80%, more than 90% of the samples of the patient are classified as "high polysomy" according to the classification criteria described before. Similarly, it is consider that exist "high LOH levels of PTEN" when more than 50%, more than 60%, more than 70%, more than 80%, more than 90% of the samples of the patient are classified as "LOH" according to the classification criteria described before.

For the generation of a table for the clarification of the patients attending to the LOH and number of copies of the PTEN gene, the person skilled in the art would, for example clarified the patients into 4 strata with ascending number of copies of the PTEN gene per cell according to the frequency of tumor cells with specific number of copies of the PTEN gene per chromosome 10. As it was mentioned before, control probes that detect the chromosome 10 for obtaining a ratio number of copies of PTEN gene/chromosome 10 are commercially available. For example the CEP 10 SpectrumGreen probe (Vysis) that hybridizes to alpha satellite sequences specific to chromosome 10, can be used.

An non limitative example of a table for classifying a patient attending to the polysomy/LOH degrees is:
1) Disomy (D): 2 copies of each probe in 90% of cells
2) LOH = <2 copies of PTEN probe in 10% of cells (includes cromosome 10 monosomy or disomy with PTEN loss, always in >10% cells)
3) Polysomy (P) = ≥ 3 copies each probe (PTEN+ CEP 10) in >10% of cells. It does not discriminate between high and low polisomy, or chromosome 10 trisomy)
4) Amplified (AMP) = defined by a ratio of PTEN gene to chromosome 10 of ≥ 2 er cell in ≥ 10% of analyzed cells.

In a last step of the method, low expression levels and/or high LOH levels of PTEN gene with respect to said reference values and high expression levels and/or high polysomy / amplification of EGFR gene are indicative of a good clinical outcome of the subject.

As a person skilled in the art understand, the samples can also be considered not appropriated for be include in any of the classification strata. Thus, in a preferred embodiment, additionally, samples sections of tissue from the patient are stained with colorants as hematoxylin and eosin (H&E) and reviewed by two or more persons skilled in the art (i.e pathologists) to assess overall tumor content, necrosis and overall quality (e.g. thermal cautery effect, fixation with morphology etc). In a preferred embodiment the samples used for determining the expression levels and/or the polysomy levels of EGFR gene and the expression levels and/or the LOH levels of PTEN gene, have at least 20%, at least 30%, at least 40%, at least 50%, at least 60%, at least 70%, at least 80%, at least 90% of tumor tissue in the sample, and have an appropriate fixation with acceptable morphology.

The findings of the inventors allow the determination of the clinical outcome of a patient suffering glioma measuring the expression levels and/or the LOH levels of PTEN gene. Thus, in a second aspect, the invention relates to a method for predicting the clinical outcome of a subject suffering from glioma comprising
(a) determining the expression levels and/or the LOH levels of PTEN gene in a sample from the subject and
(b) comparing said expression levels and/or the LOH levels of PTEN gene with standard reference values,
wherein low expression levels and/or high LOH levels of PTEN gene with respect to said reference values, is indicative of a poor clinical outcome of the subject.

The term glioma has been previously defined.

In a preferred embodiment, the glioma is a gliobastoma multiforme (GMB) and more preferably the gliobastoma is an early gliobastoma.

In a preferred embodiment, the clinical outcome is measured as survival.

The term "sample" has been previously defined and can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In a particular embodiment, said sample is a tumour tissue sample or portion thereof In a more particular embodiment, said tumor tissue sample is a brain tumor tissue sample from a patient suffering from glioma or a formalin embedded brain tissue sample.

The methods for determining the expression levels and the LOH of PTEN have been described above as well as the standard reference values used. In a preferred embodiment, the expression levels of PTEN gene are measured by determining mRNA and/or protein expression level of said gene.

In a preferred embodiment, the polysomy levels and the LOH levels are determined by FISH.

In another aspect, the invention relates to a kit useful in the implementation of the methodology described herein.

Thus, in another aspect, the invention relates to a kit comprising a set of agents capable of specifically detect the expression levels and/or the polysomy / amplification of EGFR and the expression levels and/or the LOH of PTEN genes and, optionally, a reagent for detecting a housekeeping gene or the protein encoded by said housekeeping gene and/or a reagent for detecting the chromosomes 7 and 10.

In a particular embodiment of the kit of the invention, the agents of the kit are capable of specifically detecting the mRNA levels of EGFR and/or PTEN genes or the levels of EGFR and/or PTEN proteins.

Nucleic acids capable of specifically hybridizing with the EGFR and/or PTEN genes can be one or more pairs of primer oligonucleotides for the specific amplification of fragments of the mRNAs (or of their corresponding cDNAs) of said genes and/or one or more probes for the identification of one or more genes selected from said genes. Nucleic acids capable of specifically hybridizing with the EGFR and/or PTEN genes can be as well FISH probes.

Antibodies, or a fragment thereof, capable of detecting an antigen, capable of specifically binding to EGFR and/or PTEN proteins or to variants thereof are, for example, monoclonal and polyclonal antibodies, antibody fragments, Fv, Fab, Fab' y F(ab')2, ScFv, diabodies, triabodies, tetrabodies and humanised antibodies.

Said reagents, specifically, the probes and the antibodies, may be fixed onto a solid support, such as a membrane, a plastic or a glass, optionally treated in order to facilitate fixation of said probes or antibodies onto the support. Said solid support, which comprises, at least, a set of antibodies capable of specifically binding to EGFR and/or PTEN proteins or to variants thereof, and/or probes specifically hybridized with the EGFR and/or PTEN genes, may be used for the detection of the expression levels by means of array technology.

The kits of the invention optionally comprise additional reagents for detecting a polypeptide encoded by a housekeeping gene or the mRNA encoded by said housekeeping genes. The availability of said additional reagent allows the normalization of measurements taken in different samples (e.g. the test sample and the control sample) to exclude that the differences in expression of the different biomarkers are due to a different amount of total protein in the sample rather than to real differences in relative expression levels. Housekeeping genes, as used herein, relates to genes which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin.

In another embodiment, the invention relates to the use of a kit of the invention for predicting the clinical outcome of a subject suffering from gliobastoma multiforme, wherein if said agents detect a high expression levels and/or high levels of polysomy / amplification of EGFR gene and low expression levels and/or high LOH levels of PTEN gene, with respect to y standard reference values, then the clinical outcome of the subject is high.

Methods for detecting the expression levels of EGFR and PTEN and the methods for determining the polysomy of EGFR and the LOH of PTEN as well as the standard reference values has been described previously.

In another embodiment, the invention relates to the use of EGFR expression levels and/or polysomy / amplification levels of EGFR gene and the expression levels and/or LOH levels of PTEN gene as predictive marker of the outcome of a glioma patient.

In a preferred embodiment, the glioma is a gliobastoma multiforme (GMB).

In another embodiment, the invention relates to the use of erlotinib and/or temozolomide in the manufacture of a medicament for the treatment of a glioma, wherein the medicament is for subjects having a low expression levels and/or high LOH levels of PTEN gene with respect to standard reference values and high expression levels and/or high polysomy / amplification of EGFR gene with respect to said standard reference values.

In a preferred embodiment, the glioma is a gliobastoma multiforme (GMB).

### EXAMPLES

### I. MATERIAL AND METHODS

### Patients and specimens.

Multiple formalin-fixed, paraffin-embedded blocks from fifty-six (56) primary brain tumor specimens were obtained from the Hospital Universitari de Belvitge. These patients were part of a longitudinal cohort and selected utilizing pre-determined inclusion criteria which included a primary diagnosis of any one of the following criteria: astrocytic, oligodendrocytic and glial tumor, with continuous follow-up for a median of 3 years and available tissue material. Clinical data was abstracted from the patient's clinical records utilizing a set of pre-determined clinical-pathologic and outcome data fields (see Table 1). The histologic breakdown of the tumor specimens were as follows: 1. Astrocytoma group, total 44 patients: (1) pilocytic, (13) diffuse, (16) anaplastic, and (14) glioblastoma multiforme; II. Oligodendroglioma group, total 6 patients: (6) oligodendroglioma; and **III**. Mixed, total four patient: (3) oligoastrocytoma, and one (1) anaplastic oligoastrocytoma. A comprehensive review of the available clinical data files was performed on all patients in which formalin-fixed, paraffin-embedded (FFPE) samples / blocks were available for further analysis. 5 µm sections were obtained from all blocks, stained with hematoxylin and eosin (H&E) and reviewed by two pathologists to assess overall tumor content = 50% tumor in at least one 200X field), necrosis and overall quality (e.g. thermal cautery effect, appropriate fixation with acceptable morphology etc). Subsequent sections were then obtained for DNA FISH assays and quantitative immunofluorescence as outlined below. All blocks were retained for subsequent studies including RT-PCR.

| **Table 1. Clinical data fields for entire cohort with breakdown by diagnostic group** | | **Glioblastoma** | **Astrocytoma** | **mixed/ others** | **Non available** |
|---|---|---|---|---|---|
| | | N=15 | N=26 | N=5 | N=10 |
| Patients gender: | Male | 8 | 17 | 4 | 5 |
| | Female | 7 | 9 | 1 | 3 |
| | Non available | 0 | 0 | 0 | 2 |
| Patients race | caucasian | 15 | 25 | 5 | 8 |
| | Non available | 0 | 1 | 0 | 2 |
| Symptoms seizures | Yes | 4 | 13 | 2 | 5 |
| | No | 11 | 13 | 2 | 3 |
| | Non available | 0 | 0 | 1 | 2 |
| Sypmptoms high intracranial | yes | 7 | 5 | 1 | 1 |
| pressure | No | 8 | 21 | 3 | 7 |
| | Non available | 0 | 0 | 1 | 2 |
| Number of lesions in | image 1 | 12 | 21 | 5 | 7 |
| pretreatment | Image 2 | 1 | 1 | 0 | 0 |
| | multiples | 2 | 3 | 0 | 1 |
| | Non available | 0 | 1 | 0 | 2 |
| 1 location of lesions in | frontal lobe | 10 | 10 | 3 | 5 |
| pretreatment image: | temoral lobe | 3 | 3 | 0 | 1 |
| | parietal lobe | 1 | 6 | 1 | 0 |
| | operculum | 0 | 1 | 0 | 0 |
| | brain stem | 1 | 2 | 1 | 0 |
| | spinal cord | 0 | 1 | 0 | 0 |
| | optic nerve | 0 | 0 | 0 | 1 |
| | interhemispheri | 0 | 1 | 0 | 0 |
| | c cisure | | | | |
| | cerebellum | 0 | 0 | 0 | 1 |
| | | 0 | 1 | 0 | 0 |
| | Non available | 0 | 1 | 0 | 2 |
| 2 location of lesions in | temoral lobe | 1 | 1 | 0 | 1 |
| pretreatment image | parietal lobe | 3 | 1 | 0 | 0 |
| | occipital lobe | 2 | 1 | 0 | 1 |
| | corpus callosum | 0 | 1 | 0 | 0 |
| | basal ganglia | 2 | 0 | 0 | 0 |
| | brain stem | 0 | 1 | 0 | 0 |
| | thalamus | 0 | 0 | 0 | 1 |
| | Non available | 7 | 21 | 5 | 7 |
| 1 side of lesions in pretreatment | Right | 9 | 12 | 2 | 6 |
| image | Left | 4 | 11 | 3 | 2 |
| | Unknown | 0 | 1 | 0 | 0 |
| | Non available | 2 | 2 | 0 | 2 |
| 2 side of lesions in pretreatment | Left | 1 | 1 | 0 | 1 |
| image | Non available | 14 | 25 | 5 | 9 |
| Type of specimen | biopsy | 1 | 6 | 0 | 4 |
| | stereotaxic | 2 | 4 | 0 | 1 |
| | biopsy | | | | |
| | Resection | 12 | 16 | 5 | 3 |
| | Non available | 0 | 0 | 0 | 2 |
| Type of postsurgical removal | complete | 4 | 11 | 1 | 6 |
| | Incomplete | 10 | 12 | 4 | 2 |
| | Non available | 1 | 3 | 0 | 2 |
| Radiation therapy | yes | 7 | 12 | 3 | 1 |
| | No | 8 | 12 | 2 | 7 |
| | Non available | 0 | 2 | 0 | 2 |
| Type of radiation | focal | 5 | 8 | 0 | 1 |
| | Non available | 10 | 18 | 5 | 9 |
| Total dose of radiation (* *) (Available data: n=20) | | 60.0 (60.0-60.0) | 60.0 (40.0-60.0) | 51.0 (48.0-54.0) | 60.0 (60.060.0) |
| Chemotherapy | Yes | 2 | 9 | 1 | 1 |
| | No | 13 | 15 | 4 | 7 |
| | Non available | 0 | 2 | 0 | 2 |
| Type of first chemotherapy | BCNU | 0 | 7 | 0 | 0 |
| | TMZ | 2 | 1 | 0 | 0 |
| | PCV | 0 | 1 | 0 | 1 |
| | Non available | 13 | 17 | 5 | 9 |
| Recurrence or progression | Yes | 4 | 13 | 4 | 6 |
| | No | 9 | 10 | 1 | 1 |
| | Non available | 2 | 3 | 0 | 3 |
| Type of first recurrence | focal | 3 | 8 | 4 | 6 |
| | Diffuse | 1 | 1 | 0 | 0 |
| | Multiple | 0 | 2 | 0 | 0 |
| | Non available | 11 | 15 | 1 | 4 |
| Treatment of first recurrence | Yes | 3 | 10 | 4 | 5 |
| | No | 1 | 1 | 0 | 1 |
| | Non available | 11 | 15 | 1 | 4 |
| 1 type of treatment of first | Radiation | 0 | 2 | 1 | 1 |
| recurrence | Chemotherapy | 2 | 3 | 1 | 0 |
| | Surgery | 1 | 4 | 2 | 4 |
| | Radiosurgery | 0 | 1 | 0 | 0 |
| | Non available | 12 | 16 | 1 | 5 |
| 2 type of treatment of first | Radiation | 0 | 2 | 0 | 4 |
| recurrence | Chemotherapy | 0 | 1 | 0 | 0 |
| | Non available | 15 | 23 | 5 | 6 |
| 3 type of treatment of first | Chemotherapy | 0 | 1 | 0 | 1 |
| recurrence | Non available | 15 | 25 | 5 | 9 |
| Kamovsky value pretreatment | | 70.0 (40.0- | 90.0 (20.0- | 95.0 | 100 (60.0- |
| (**) (Available data: n=49) | | 100) | 100) | (90.0-100) | 100) |
| Kamovsky value postreatment | | 60.0 (30.0- | 80.0 (10.0- | 95.0 | 100 (30.0- |
| (**) (Available data: n=46) | | 90.0) | 100) | (80.0-100) | 100) |
| Death of patient | yes | 15 | 15 | 1 | 1 |
| | No | 0 | 7 | 3 | 5 |
| | Non available | 0 | 4 | 1 | 4 |

### DNA EGFR and PTEN FISH

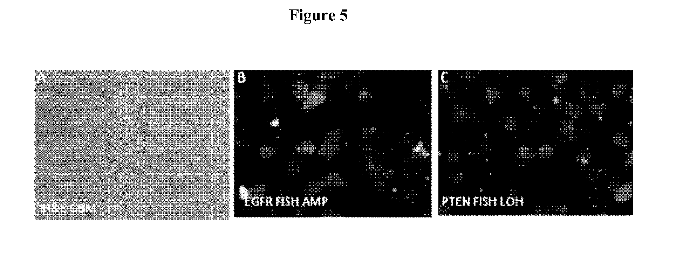

Using the LSI EGFR/CEP 7 dual color probe (Vysis, Abbot Molecular) for EGFR and the LSI PTEN (10q23) / CEP 10 dual color probe for PTEN (Vysis, Abbot Molecular), DNA FISH including hybridization, washing and fluorescence detection was performed on all specimens in the cohort as per standard protocols (Smith et al., 2001. JNCI; 93:1246-1256). Briefly, paraffin sections were dewaxed in xylenes, microwaved in 10mmol/L sodium citrate (pH 6.0) solution for 5-10 minutes, cooled to room temperature, rinsed, and then treated with pepsin HCL for 5 minutes at 37C before being rinsed and dehydrated. The prewarmed probe mixture was applied to the slides, and a cover-slip sealed in place with rubber cement. The slides were then denatured at 85°C for 4 to 6 minutes using an automated hybridization chamber and then incubated overnight at 37C. After standard post hybridization washes the slides were stained with the DNA stain DAPI and mounted with antifade (Vectashield). Prior to imaging all H&E slides were evaluated by a pathologist and regions of interest were identified based on histopathologic and quality criteria including tumor content, appropriate fixation, necrosis and vascularity. A H&E image of individual GBM cases is illustrated in Figure 5A. All slides were imaged using a Nikon immunofluorescent microscope by a trained scientist blinded to outcome. Criteria for FISH anomalies were defined by use of histologically normal brain specimens. Simple gain required 10% or more of nuclei with three or more locus-specific probe signals. Loss of the q arm of chromosome 10 required the overall mean PTEN / CEP10 ratio to be less than 0.90. Amplification was applied for both EGFR and PTEN and required that the ratio must be 2 or more and that 10% or more of nuclei had more than three EGFR and or PTEN signals. Representative regions of interest were acquired for documentation of signal in all specimens (Examples of representative images of FISH AMP/HP of EGFR and monosomy of PTEN are shown in figure 5B and C respectively). For EGFR FISH we followed the Cappuzzo F, et al. (JNCI 2005;4: 643-55) classification scheme and identified 6 categories for EGFR status in all tumor specimens. These individual states included: D=disomy, HT=high trisomy, LT=low trisomy, HP=high polysomy, LP=low polysomy, and AMP=amplification. In this schema both HP and AMP together are considered amplification. PTEN status was characterized as D=disomy, LOH=>10%, P=polysomy, AMP=amplified and NE=not evaluable. All cases were reviewed by a single observer and recorded anonymously with respect to tumor type or outcome.

### Quantitative Immunofluorescence (IF)

Utilizing previous established methods (Cordon Cardo et al., 2007 JCI 2007;117;1876-83; Donovan et al., 2008 JCO;26:3923-3929) the multiplex- 1 (mplex1) assay was constructed incorporating the following antibodies: GFAP (glial fibrillary acidic protein), PTEN (phosphatase and tensin homolog), PAKT and Ki67 combined with the nuclear stain DAPI (4',6-diamidino-2-phenylindole) - see table 2 for a complete review of antibodies used within the multiplex1 assay. The individual antibodies were evaluated in simplex IF assays using control tissues and cell lines to confirm expression sensitivity and specificity prior to inclusion in the multiplex assay.

**Table 2. Reagent list.**

| Antibody | Vendor | Catalog# | Dilution | lsotype | Labels | samples |
|---|---|---|---|---|---|---|
| | | | 1:30 | MIgG1 | 2X M 488 | GBM cases, NL brain, LNCaP, |
| PTEN | Neomarkers | | | | | Tonsil |
| Ki67 | Dako | | 1:100 | MIgG1 | 2X M 555 | same |
| pAKT | CST | #3787 | 1:25 | RIgG | 2X R 594 | same |
| GFAP | Dako | | 1-200 | MIgG1 | 2X M 647 | same |

In brief, FFPE samples were de-paraffinized, rehydrated and subjected to an antigen retrieval process with the Reveal buffer system (Biocare Medical). A series of four antibodies were combined with DAPI into a multiplex immunofluorescent assay. The reagents listed in Table 2 were differentially labeled and with the identified fluorochromea and then sequentially applied onto individual sections using a Nemesis 7200 immunostainer (BioCare Medical). A minimum of three images or regions of interest (ROI) were acquired by a pathologist (blinded to outcome) using a Nikon 90i immunofluorescent microscope equipped with a CRI spectral imaging system (CRI). Utilizing pre-developed unmixing libraries and CRI software, individual gray scale images were generated which represent the antibody - filter combination under investigation. The individual images are evaluated for signal:noise ratio, cellular distribution and co-distribution with other antibody-fluorochrome labeled reagents. Using the existing CRI software manual thresholds were created for individual antibody-fluorochrome combinations to maximize signal:noise and preserving distribution. Quantitative metrics were generated using the software to identify percentages of individual cell populations exhibiting a positive signal (based on the threshold), normalized to the tumor region under evaluation. A series of prostate cancer cell lines including LNCaP, PC3 and DU145 (ATCC) were obtained, grown to confluency, harvested as agar cell pellets, fixed and embedded in paraffin and then all three cell lines were placed into a cell array for quality control both during assay development and investigation of brain tumor cases.

### Results and Discussion

### Patients and specimens

There were 54 patients with available clinical records and FFPE tissue samples appropriate for evaluation. The breakdown of patients is listed in Table 3 with the majority of patients in the astrocytoma diagnostic category. The individual demographic features including sex, location of mass, treatment and outcome data are outlined in detail in Table 1.

**Table 3. Diagnosis category of the patients.**

| Original codes | Pure GBM | Astrocytoma | Oligodendroglioma | Mixed/ other |
|---|---|---|---|---|
| 001: astrocytoma grade II | 0 | **10** | 0 | 0 |
| 002: astrocytoma grade III | 0 | **16** | 0 | 0 |
| 003: glioblastoma | **15** | 0 | 0 | 0 |
| 004: oligoastroctoma grade II | 0 | 0 | 0 | **3** |
| 005: oligoastrocytoma grade III | 0 | 0 | 0 | **2** |
| 006: oligodendroglioma grade II | 0 | 0 | **4** | 0 |
| 007: oligodendroglioma gradeIII | 0 | 0 | **1** | 0 |
| 008: pilocytic, astrocytoma | 0 | 2 | 0 | 0 |
| 009: gliomatosis cerebri 999: Non available | 0 | 0 | 0 | **1** |
| 999 : Not available | 0 | 0 | 0 | 2 |

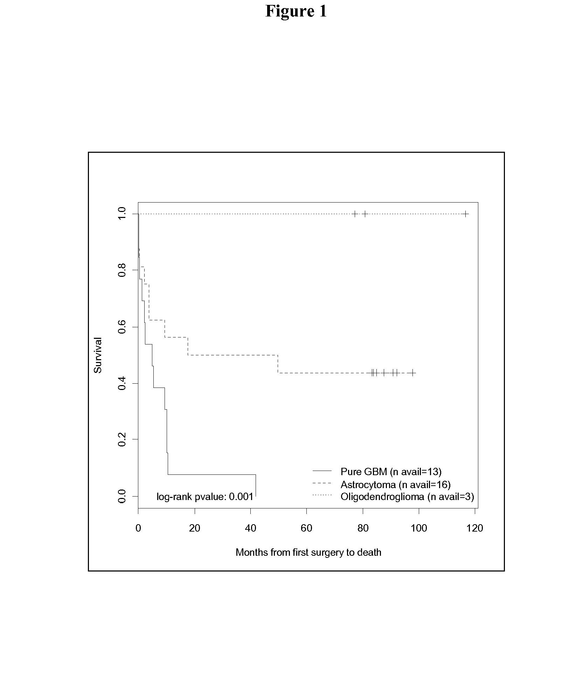

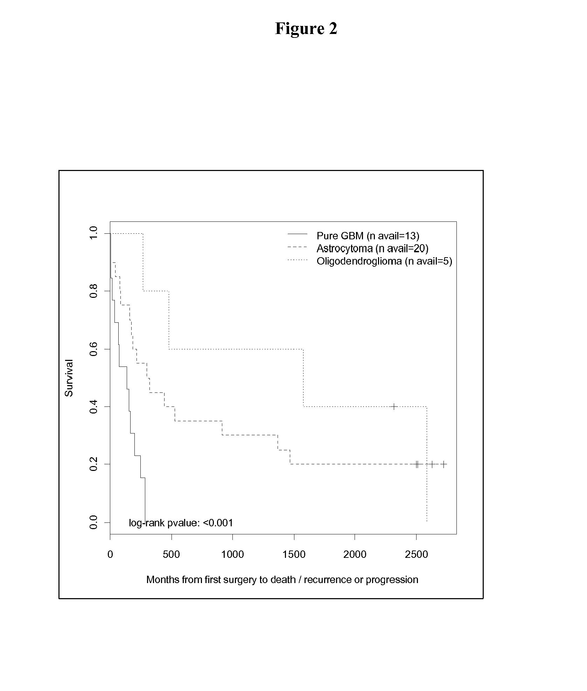

In order to understand general survival trends within the cohort and in particular the glioblastoma group vs. others we performed Kaplan-Meier survival function analyses to estimate survival between the three groups. Figure 1 is the Kaplan-Meier survival curve demonstrating reduced survival in the glioblastoma group vs. the anaplastic astrocytoma and mixed categories (log-rank test P=0.001). Figure 2 is a second Kaplan-Meier survival function curve which evaluates a more composite end-point which includes progression free and overall survival in the same three diagnostic categories (log-rank test P<0.001). In agreement with the literature, the glioblastoma group has a reduced overall and progression free survival when compared to the astrocytoma and pure oligodendroglioma group which has the longest survival time of all three diagnostic categories. As evident from the curves, a subset of the anaplastic astrocytoma group appears to behave like the glioblastoma category.

### EGFR and PTEN FISH

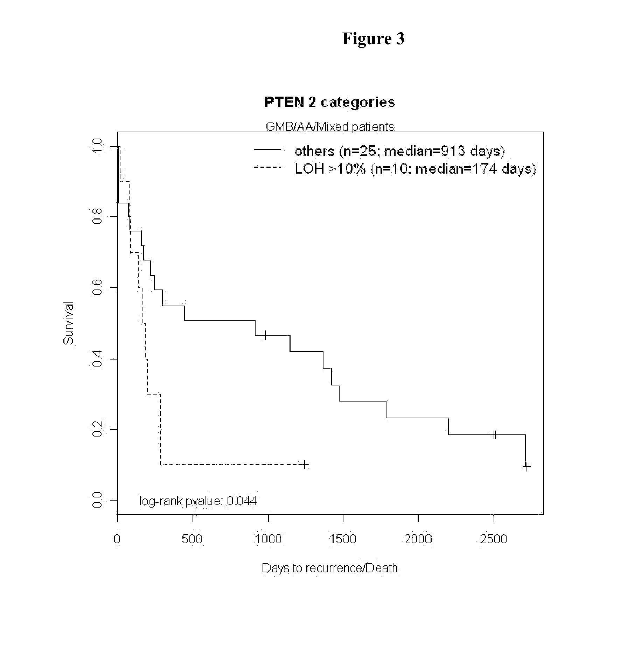

Given the frequency of EGFR amplification (approximately 40%) and reported role that EGFR plays in the development of glioblastoma, the first DNA FISH study performed was on the characterization of EGFR. Using the scoring methods of both Cappuzzo et al., 2005 JNCI;4:643-55and Smith et al., 2001 JNCI;93:1246-1256, we investigated EGFR profiles in all brain tumor cases in the group. Given the overall cohort size we opted to further classify patients as positive if they exhibited either polysomy and/or amplification of the EGFR locus or chromosome 7. This is in contrast to some reported studies which independently characterize EGFR DNA FISH as either polysomy or amplification. In our study 71% of the GBM patients had EGFR polysomy / amplification compared with 45% in the anaplastic astrocytoma group. We did not find EGFR amplification or polysomy to be associated with survival when examining the entire cohort or individual subgroups (all patients log-rank test p=0.8; glioblastoma only patients p=0.1). Dual-color FISH was also performed with probes for PTEN utilizing a similar scoring system as EGFR; however in addition to disomy and polysomy we included the loss of PTEN as 10% in tumor epithelial nuclei. In our studies 61% of GBM samples had PTEN LOH compared with 25% in the anaplastic astrocytoma group. There is sparse literature on the evaluation of PTEN by DNA FISH in brain tumor samples with most studies using more molecular techniques such as sequencing, RT-PCR and immunohistochemistry. Noteworthy in our cohort, PTEN LOH was univariately and statistically associated with reduced survival in the combined glioblastoma, astrocytoma and mixed patient group (n=25 without LOH, median survival 913D vs. n=10 with LOH, median survival 174D; log-rank test p=0.04) See figure 3.

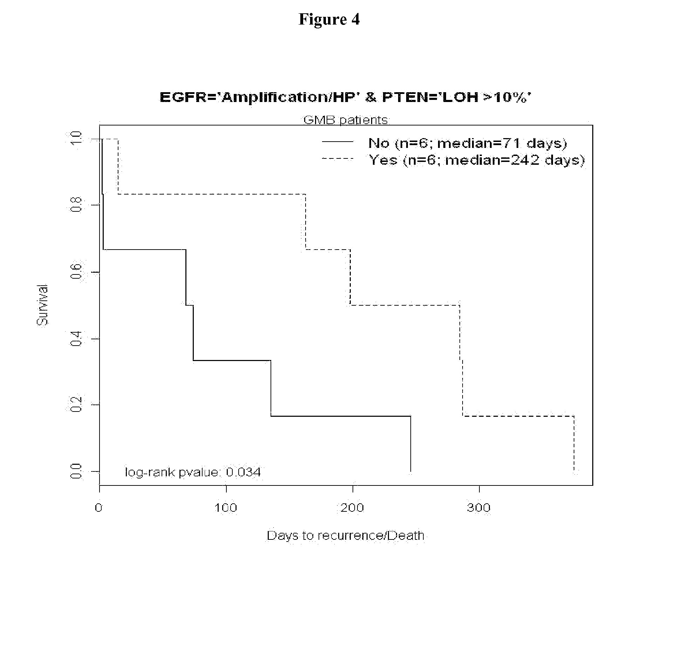

Neither EGFR polysomy / amplification or PTEN loss were significant, independent predictors for survival in the glioblastoma only group (p=0.1); however, when combined we observed a trend towards significance with an increase in overall survival (n=6 with both EGFR/PTEN, median survival 242D vs. n=6 without, median survival 71D; log-rank test p=0.034) (see Figure 4) The hypothesis is that the combination of PTEN LOH and EGFR amplification in glioblastoma may represent a subset of patients with a tumor phenotype more amenable to radiation and/ or temozolomide treatment.

**Tables 4.Clinical characteristics of patients with previous filter conditions.**

| Idalthia | gender | age | sympseiz | sympticp. | imagelesions | imagelocat_1 | imagelocat_2 | imageside_1 | imageside_2 | diagn |
|---|---|---|---|---|---|---|---|---|---|---|
| L08-00080 | female | 50 | no | yes | 1 | frontal lobe | NA | left | NA, | glioblastoma |
| L06-00084 | female | 65 | no | yes | 1 | frontal lobe | NA | right | NA. | glioblastoma |
| L08-00035 | male | 71 | no | yes | 1 | temporal lobe | NA | right | NA | glioblastoma |
| L08-00087 | male. | 66 | no | no | 1 | frontal lobe | parietal lobe | right | NA | glioblastoma |
| L08-00090 | female | 74 | no | no | 1 | parietal lobe | occipital lobe | right | NA | glioblastoma |
| L08-00091 | male | 68 | no | no | 1 | temporal lobe | NA | right | NA | glioblastoma |
| L08-00094 | female | 66 | yes | no | 1 | frontal lobe | temporal lobe | right | NA | glioblastoma |
| L08-00097 | male | 50 | yes | yes | 1 | temporal lobe | NA | right | NA | glioblastoma |
| L08-00098 | female | 49 | yes | no | 1 | frontal lobe | parietal lobe | left | NA | glioblastoma |
| L08-00106, | male | 66 | yes | yes | multiples | frontal lobe | basal ganglia | NA | NA | glioblastoma |
| L08-00109 | female | 60 | no | no | 2 | frontal lobe | occipital lobe | left | NA | glioblastoma |
| L08-00128 | male | 37 | no | yes | 1 | frontal lobe | NA | right | left | glioblastoma |

| | | | | | | | | | | |
|---|---|---|---|---|---|---|---|---|---|---|
| MA: data non-available | | | | | | | | | | |

**table4.**

| Idalthia | diagnspec | psiremoval | recur progr | recurtype | death |
|---|---|---|---|---|---|
| L09-00080 | resection | complete | yes | NA | yes |
| L08-00084 | resection | compete | yes | focal | yes |
| L08-00085 | resection | complete | yes | focal | yes |
| L08-00087 | resection | incomplete | no | NA | yes |
| L08-00090 | biopsy | incomplete | no | NA | yes |
| L09-00091 | resection | complete | no | NA | yes |
| L08-00084 | resection | NA | no | NA | yes |
| L08-00097 | resection | incomplete | no | NA | yes |
| L08-00098 | resection | incomplete | yes | focal | yes |
| L08-00106 | stereothaxic biopsy | incomplete | no | NA | yes |
| L08-00108 | stereothaxic biopsy | incomplete | no | NA | yes |
| L08-00128 | resection | incomplete | yes | diffuse | yes |

| | | | | | |
|---|---|---|---|---|---|
| NA: data non-available | | | | | |

VARIABLE INFO Table 4:
gender = "patients gender"
age = "patients age"
sympseiz = "symptoms seizures"
sympticp = "sypmptoms high intracranial pressure"
imagelesions = "number of lesions in pretreatment image"
imagelocat_1 = "location of 1st. lesion in pretreatment image"
imagelocat_2 = "location of 2nd. lesion in pretreatment image"
imageside_1 = "side of 1st. lesion in pretreatment image"
imageside_2 = "side of 2nd. lesion in pretreatment image"
diagn = "pathological diagnosis"
diagnspec = "type of specimen"
psiremoval = "type of postsurgical removal"
recur.progr = "recurrence or progression"
recurrtype = "type of first recurrence"
death = "death of patient"

In table 4 are included 12 patients, 6 with the EGFR AMP / PTEN LOH phenotype and 6 without (see column 2, EGFR/PTEN status (yes / no with respect to phenotype). Of the 6 Glioblastoma multiforme (GBM) patients with this phenotype, 3 exhibited no evidence of clinical recurrence. The 3 patients who did recur in the EGFR AMP / PTEN LOH positive group had received radiation and one received both radiation and the chemotherapeutic agent temozolomide (TMZ). Although the number of patients is small the data suggests that GBM patients with a tumor exhibiting the EGFR AMP / PTEN LOH have a better overall clinical outcome than patients without this phenotype regardless of current treatment including radiation and chemotherapy. We conclude that the identified phenotype which involves both EGFR signaling and PTEN loss may reflect an overall good acting GBM, irrespective of current treatment modalities and that newer investigative therapies and clinical trials would benefit from focusing on the intersect of these two pathways to further improve outcome.

## Claims

1. Method for predicting the clinical outcome of a subject suffering from glioma that comprises
a) determining the expression levels or the polysomy / amplification levels of EGFR gene and the expression levels and/or the LOH levels of PTEN gene in a sample from the subject and
b) comparing said expression levels or the polysomy / amplification levels of EGFR gene and the expression levels and/or the LOH levels of PTEN gene with standard reference values,
wherein low expression levels and/or high LOH levels of PTEN gene with respect to said standard reference values and high expression levels and/or high polysomy / amplification of EGFR gene with respect to said standard reference values are indicative of a good clinical outcome of the subject.

2. Method for predicting the clinical outcome of a subject suffering from glioma comprising
(a) determining the expression levels and/or the LOH levels of PTEN gene in a sample from the subject and
(b) comparing said expression levels and/or the LOH levels of PTEN gene
with standard reference values,
wherein low expression levels and/or high LOH levels of PTEN gene with respect to said standard reference values, is indicative of a poor clinical outcome of the subject.

3. Method according to claim 1, wherein the glioma is a gliobastoma multiforme (GBM).

4. Method according to claim 1-3, wherein the clinical outcome is measured as survival.

5. Method according to claims 1-4, wherein the sample is a tumor tissue sample.

6. Method according to any of claims 1-5, wherein the expression levels of EGFR and the expression levels of PTEN gene are measured by determining mRNA and/or protein expression levels of said genes.

7. Method according to claim 5, wherein the polysomy levels and the LOH levels are determined by FISH.

8. Method according to any of claims 1 to 9, wherein the gliobastoma is early gliobastoma.

9. Kit comprising a set of agents capable of specifically detect the expression levels and/or the polysomy / amplification of EGFR and the expression levels and/or the LOH of PTEN genes and, optionally, a reagent for detecting a housekeeping gene or the protein encoded by said housekeeping gene and/or a reagent for detecting the chromosomes 7 and 10.

10. Kit according to claim 9, wherein the agents of the kit are capable of specifically detecting the mRNA levels of EGFR and/or PTEN genes or the levels of EGFR and/or PTEN proteins.

11. Kit according to claim 9-10, wherein the reagents of the kit are DNA or RNA probes and antibodies.

12. Use of a kit according to anyone of claims 9 and 11 for predicting the clinical outcome of a subject suffering from gliobastoma multiforme, wherein if said agents detect a high expression levels and/or high levels of polysomy / amplification of EGFR gene and low expression levels and/or high LOH levels of PTEN gene, with respect to reference values, then the clinical outcome of the subject is high.

13. Use of both EGFR expression levels and/or polysomy / amplification levels of EGFR gene and expression levels and/or LOH levels of PTEN gene as predictive marker of the outcome of a glioma patient.

14. Use of erlotinib and/or temozolomide and /or radiotherapy in the manufacture of a medicament for the treatment of a glioma, wherein the medicament is for subjects having a low expression levels and/or high LOH levels of PTEN gene with respect to standard reference values and high expression levels and/or high polysomy / amplification of EGFR gene with respect to said standard reference values.

15. Use of erlotinib and/or temozolomide and /or radiotherapy according to claim 14, wherein the glioma is a gliobastoma multiforme (GBM).


SEQUENCE LISTING

<110>  Europath biosciences S.L.

<120>  EGFR AND PTEN GENE ALTERATIONS PREDICTS SURVIVAL IN PATIENTS WITH
        BRAIN TUMORS

<130>  P5001EP00

<160>  13

<170>  PatentIn version 3.5

<210>  1
<211>  5572
<212>  DNA
<213>  Homo sapiens

<400>  1
cctcccctcg cccggcgcgg tcccgtccgc ctctcgctcg cctcccgcct cccctcggtc      60

ttccgaggcg cccgggctcc cggcgcggcg gcggaggggg cgggcaggcc ggcgggcggt     120

gatgtggcgg gactctttat gcgctgcggc aggatacgcg ctcggcgctg ggacgcgact     180

gcgctcagtt ctctcctctc ggaagctgca gccatgatgg aagtttgaga gttgagccgc     240

tgtgaggcga ggccgggctc aggcgaggga gatgagagac ggcggcggcc gcggcccgga     300

gcccctctca gcgcctgtga gcagccgcgg gggcagcgcc ctcggggagc cggccggcct     360

gcggcggcgg cagcggcggc gtttctcgcc tcctcttcgt cttttctaac cgtgcagcct     420

cttcctcggc ttctcctgaa agggaaggtg gaagccgtgg gctcgggcgg gagccggctg     480

aggcgcggcg gcggcggcgg cacctcccgc tcctggagcg ggggggagaa gcggcggcgg     540

cggcggccgc ggcggctgca gctccaggga gggggtctga gtcgcctgtc accatttcca     600

gggctgggaa cgccggagag ttggtctctc cccttctact gcctccaaca cggcggcggc     660

ggcggcggca catccaggga cccgggccgg ttttaaacct cccgtccgcc gccgccgcac     720

cccccgtggc ccgggctccg gaggccgccg gcggaggcag ccgttcggag gattattcgt     780

cttctcccca ttccgctgcc gccgctgcca ggcctctggc tgctgaggag aagcaggccc     840

agtcgctgca accatccagc agccgccgca gcagccatta cccggctgcg gtccagagcc     900

aagcggcggc agagcgaggg gcatcagcta ccgccaagtc cagagccatt tccatcctgc     960

agaagaagcc ccgccaccag cagcttctgc catctctctc ctcctttttc ttcagccaca    1020

ggctcccaga catgacagcc atcatcaaag agatcgttag cagaaacaaa aggagatatc    1080

aagaggatgg attcgactta gacttgacct atatttatcc aaacattatt gctatgggat    1140

ttcctgcaga aagacttgaa ggcgtataca ggaacaatat tgatgatgta gtaaggtttt    1200

tggattcaaa gcataaaaac cattacaaga tatacaatct ttgtgctgaa agacattatg 1260
acaccgccaa atttaattgc agagttgcac aatatccttt tgaagaccat aacccaccac 1320
agctagaact tatcaaaccc ttttgtgaag atcttgacca atggctaagt gaagatgaca 1380
atcatgttgc agcaattcac tgtaaagctg gaaagggacg aactggtgta atgatatgtg 1440
catatttatt acatcggggc aaatttttaa aggcacaaga ggccctagat ttctatgggg 1500
aagtaaggac cagagacaaa aagggagtaa ctattcccag tcagaggcgc tatgtgtatt 1560
attatagcta cctgttaaag aatcatctgg attatagacc agtggcactg ttgtttcaca 1620
agatgatgtt tgaaactatt ccaatgttca gtggcggaac ttgcaatcct cagtttgtgg 1680
tctgccagct aaaggtgaag atatattcct ccaattcagg acccacacga cgggaagaca 1740
agttcatgta ctttgagttc cctcagccgt tacctgtgtg tggtgatatc aaagtagagt 1800
tcttccacaa acagaacaag atgctaaaaa aggacaaaat gtttcacttt tgggtaaata 1860
cattcttcat accaggacca gaggaaacct cagaaaaagt agaaaatgga agtctatgtg 1920
atcaagaaat cgatagcatt tgcagtatag agcgtgcaga taatgacaag gaatatctag 1980
tacttacttt aacaaaaaat gatcttgaca aagcaaataa agacaaagcc aaccgatact 2040
tttctccaaa ttttaaggtg aagctgtact tcacaaaaac agtagaggag ccgtcaaatc 2100
cagaggctag cagttcaact tctgtaacac cagatgttag tgacaatgaa cctgatcatt 2160
atagatattc tgacaccact gactctgatc cagagaatga accttttgat gaagatcagc 2220
atacacaaat tacaaaagtc tgaatttttt tttatcaaga gggataaaac accatgaaaa 2280
taaacttgaa taaactgaaa atggaccttt ttttttttaa tggcaatagg acattgtgtc 2340
agattaccag ttataggaac aattctcttt tcctgaccaa tcttgtttta ccctatacat 2400
ccacagggtt ttgacacttg ttgtccagtt gaaaaaaggt tgtgtagctg tgtcatgtat 2460
ataccttttt gtgtcaaaag gacatttaaa attcaattag gattaataaa gatggcactt 2520
tcccgtttta ttccagtttt ataaaaagtg gagacagact gatgtgtata cgtaggaatt 2580
ttttcctttt gtgttctgtc accaactgaa gtggctaaag agctttgtga tatactggtt 2640
cacatcctac ccctttgcac ttgtggcaac agataagttt gcagttggct aagagaggtt 2700
tccgaagggt tttgctacat tctaatgcat gtattcgggt taggggaatg gagggaatgc 2760
tcagaaagga aataatttta tgctggactc tggaccatat accatctcca gctatttaca 2820
cacacctttc tttagcatgc tacagttatt aatctggaca ttcgaggaat tggccgctgt 2880
cactgcttgt tgtttgcgca ttttttttta aagcatattg gtgctagaaa aggcagctaa 2940
aggaagtgaa tctgtattgg ggtacaggaa tgaaccttct gcaacatctt aagatccaca 3000

aatgaaggga tataaaaata atgtcatagg taagaaacac agcaacaatg acttaaccat 3060

ataaatgtgg aggctatcaa caaagaatgg gcttgaaaca ttataaaaat tgacaatgat 3120

ttattaaata tgttttctca attgtaacga cttctccatc tcctgtgtaa tcaaggccag 3180

tgctaaaatt cagatgctgt tagtacctac atcagtcaac aacttacact tattttacta 3240

gttttcaatc ataatacctg ctgtggatgc ttcatgtgct gcctgcaagc ttcttttttc 3300

tcattaaata taaaatattt tgtaatgctg cacagaaatt ttcaatttga gattctacag 3360

taagcgtttt ttttctttga agatttatga tgcacttatt caatagctgt cagccgttcc 3420

acccttttga ccttacacat tctattacaa tgaattttgc agttttgcac attttttaaa 3480

tgtcattaac tgttagggaa ttttacttga atactgaata catataatgt ttatattaaa 3540

aaggacattt gtgttaaaaa ggaaattaga gttgcagtaa actttcaatg ctgcacacaa 3600

aaaaaagaca tttgattttt cagtagaaat tgtcctacat gtgctttatt gatttgctat 3660

tgaaagaata gggttttttt tttttttttt tttttttttt ttaaatgtgc agtgttgaat 3720

catttcttca tagtgctccc ccgagttggg actagggctt caatttcact tcttaaaaaa 3780

aatcatcata tatttgatat gcccagactg catacgattt taagcggagt acaactacta 3840

ttgtaaagct aatgtgaaga tattattaaa aaggtttttt tttccagaaa tttggtgtct 3900

tcaaattata ccttcacctt gacatttgaa tatccagcca ttttgtttct taatggtata 3960

aaattccatt ttcaataact tattggtgct gaaattgttc actagctgtg gtctgaccta 4020

gttaatttac aaatacagat tgaataggac ctactagagc agcatttata gagtttgatg 4080

gcaaatagat taggcagaac ttcatctaaa atattcttag taaataatgt tgacacgttt 4140

tccatacctt gtcagtttca ttcaacaatt tttaaatttt taacaaagct cttaggattt 4200

acacatttat atttaaacat tgatatatag agtattgatt gattgctcat aagttaaatt 4260

ggtaaagtta gagacaacta ttctaacacc tcaccattga aatttatatg ccaccttgtc 4320

tttcataaaa gctgaaaatt gttacctaaa atgaaaatca acttcatgtt ttgaagatag 4380

ttataaatat tgttctttgt tacaatttcg ggcaccgcat attaaaacgt aactttattg 4440

ttccaatatg taacatggag ggccaggtca taaataatga cattataatg ggcttttgca 4500

ctgttattat ttttcctttg gaatgtgaag gtctgaatga gggttttgat tttgaatgtt 4560

tcaatgtttt tgagaagcct tgcttacatt ttatggtgta gtcattggaa atggaaaaat 4620

ggcattatat atattatata tataaatata tattatacat actctcctta ctttatttca 4680

gttaccatcc ccatagaatt tgacaagaat tgctatgact gaaaggtttt cgagtcctaa 4740

ttaaaacttt atttatggca gtattcataa ttagcctgaa atgcattctg taggtaatct 4800

ctgagtttct ggaatatttt cttagacttt ttggatgtgc agcagcttac atgtctgaag 4860

ttacttgaag gcatcacttt taagaaagct tacagttggg ccctgtacca tcccaagtcc 4920

tttgtagctc ctcttgaaca tgtttgccat acttttaaaa gggtagttga ataaatagca 4980

tcaccattct ttgctgtggc acaggttata aacttaagtg gagtttaccg gcagcatcaa 5040

atgtttcagc tttaaaaaat aaaagtaggg tacaagttta atgtttagtt ctagaaattt 5100

tgtgcaatat gttcataacg atggctgtgg ttgccacaaa gtgcctcgtt tacctttaaa 5160

tactgttaat gtgtcatgca tgcagatgga aggggtggaa ctgtgcacta aagtgggggc 5220

tttaactgta gtatttggca gagttgcctt ctacctgcca gttcaaaagt tcaacctgtt 5280

ttcatataga atatatatac taaaaaattt cagtctgtta aacagcctta ctctgattca 5340

gcctcttcag atactcttgt gctgtgcagc agtggctctg tgtgtaaatg ctatgcactg 5400

aggatacaca aaaataccaa tatgatgtgt acaggataat gcctcatccc aatcagatgt 5460

ccatttgtta ttgtgtttgt taacaaccct ttatctctta gtgttataaa ctccacttaa 5520

aactgattaa agtctcattc ttgtcaaaaa aaaaaaaaaa aaaaaaaaaa aa 5572

<210> 2
<211> 403
<212> PRT
<213> Homo sapiens

<400> 2

<210>  3
<211>  8229
<212>  DNA
<213>  Mus musculus

<400>  3
gcggcaggat acgcgcttgg gcgtcgggac gcggctgcgc tcagctctct cctctcggaa      60

gctgcagcca tgatggaagt ttgagagttg agccgctgtg aggccaggcc cggcgcaggc     120

gagggagatg agagacggcg gcggccacgg cccagagccc ctctcagcgc ctgtgagcag     180

ccgcgggggc agcgccctcg gggagccggc cgggcggcgg cggcggcagc ggcggcgggc     240

ctcgcctcct cgtcgtctgt tctaaccggg cagcttctga gcagcttcgg agagagacgg     300

tggaagaagc cgtgggctcg agcgggagcc ggcgcaggct cggcggctgc acctcccgct     360

cctggagcgg gggggagaag cggcggcggc ggccgcggct ccggggaggg ggtcggagtc     420

gcctgtcacc attgccaggg ctgggaacgc cggagagttg ctctctcccc ttctcctgcc     480

tccaacacgg cggcggcggc ggcggcacgt ccagggaccc gggccggtgt taagcctccc     540

gtccgccgcc gccgcacccc ccctggcccg ggctccggag gccgccggag gaggcagccg     600

ctgcgaggat tatccgtctt ctccccattc cgctgcctcg gctgccaggc ctctggctgc     660

tgaggagaag caggcccagt ctctgcaacc atccagcagc cgccgcagca gccattaccc     720

ggctgcggtc cagggccaag cggcagcaga gcgaggggca tcagcgaccg ccaagtccag     780

agccatttcc atcctgcaga agaagcctcg ccaccagcag cttctgccat ctctctcctc     840

ctttttcttc agccacaggc tcccagacat gacagccatc atcaaagaga tcgttagcag     900

aaacaaaagg agatatcaag aggatggatt cgacttagac ttgacctata tttatccaaa     960

tattattgct atgggatttc ctgcagaaag acttgaaggt gtatacagga acaatattga    1020

tgatgtagta aggtttttgg attcaaagca taaaaaccat tacaagatat acaatctatg    1080

tgctgagaga cattatgaca ccgccaaatt taactgcaga gttgcacagt atccttttga 1140

agaccataac ccaccacagc tagaacttat caaacccttc tgtgaagatc ttgaccaatg 1200

gctaagtgaa gatgacaatc atgttgcagc aattcactgt aaagctggaa agggacggac 1260

tggtgtaatg atttgtgcat atttattgca tcggggcaaa tttttaaagg cacaagaggc 1320

cctagatttt tatggggaag taaggaccag agacaaaaag ggagtcacaa ttcccagtca 1380

gaggcgctat gtatattatt atagctacct gctaaaaaat cacctggatt acagacccgt 1440

ggcactgctg tttcacaaga tgatgtttga aactattcca atgttcagtg gcggaacttg 1500

caatcctcag tttgtggtct gccagctaaa ggtgaagata tattcctcca attcaggacc 1560

cacgcggcgg gaggacaagt tcatgtactt tgagttccct cagccattgc ctgtgtgtgg 1620

tgatatcaaa gtagagttct tccacaaaca gaacaagatg ctcaaaaagg acaaaatgtt 1680

tcacttttgg gtaaatacgt tcttcatacc aggaccagag gaaacctcag aaaaagtgga 1740

aaatggaagt ctttgtgatc aggaaatcga tagcatttgc agtatagagc gtgcagataa 1800

tgacaaggag tatcttgtac tcaccctaac aaaaaacgat cttgacaaag caaacaaaga 1860

caaggccaac cgatacttct ctccaaattt taaggtgaaa ctatacttta caaaaacagt 1920

agaggagcca tcaaatccag aggctagcag ttcaacttct gtgactccag atgttagtga 1980

caatgaacct gatcattata gatattctga caccactgac tctgatccag agaatgaacc 2040

ttttgatgaa gatcagcatt cacaaattac aaaagtctga tttttttttt cttatcaaga 2100

gggataaaat accatgaaaa aaaaaaaact tgaataaact gaaatggacc tttttttttt 2160

tttttttttt ttaaatggca ataggacatt gtgtcagatt gcagttatag gaacaattct 2220

cttctcctga ccaatcttgt tttaccctat acatccacag ggttttgaca cttgttgtcc 2280

agttaaaaaa aggttgtgta gctgtgtcat gtatatacct ttttgtgtca aaaggacatt 2340

taaaattcaa ttaggataaa taaaagatgg cactttccca ttttattcca gttttataaa 2400

aagtggagac aggctgatgt gtatacgcag gagtttttcc tttattttct gtcaccagct 2460

gaagtggctg aagagctctg attcccgggt tcacgtccta cccctttgca cttgtggcaa 2520

cagataagtt tgcagttggc taaggaagtt tctgcagggt tttgttagat tctaatgcat 2580

gcacttgggt tgggaatgga gggaatgctc agaaaggaat gtttctacct gggctctgga 2640

ccatacacca tctccagctc cttagatgca cctttcttta gcatgctcca cttactaatc 2700

tggacatccg agagattggc tgctgtcctg ctgtttgttt gtgcatttta aagagcatat 2760

tggtgctaga caaggcagct agagtgagta tatttgtagt ggggtacagg aatgaaccat 2820

ctacagcatc ttaagaatcc acaaaggaag ggatataaaa aaagtggtca tagatagata 2880

aaagacacag cagcaatgac ttaaccatac aaatgtggag gctttcaaca aaggatgggc 2940

tggaaacaga aaatttgaca atgatttatt cagtatgctt tctcagttgt aatgactgct 3000

ccatctccta tgtaatcaag gccagtgcta agagtcagat gctattagtc cctacatcag 3060

tcaacacctt acctttattt ttattaattt tcaatcatat acctactgtg gatgcttcat 3120

gtgctggctg ccagtttgtt tttctcctta aatattttat aattcttcac aggaaatttc 3180

aacttgagat tcaacagtaa gcaggttttg tttttttttt ttcctagaga ttgatgatgc 3240

gcgtcctcag tccagtggct gtcagacgtt cagccccttt gaccttacac attctattac 3300

aatgagtttt gcagttttgc acattttttt taaatgtcat taactgttag ggaattttac 3360

ttgaatactg aatacatata atgtgtatat taaaaaagtc attgtttgtg ttaaaaaaga 3420

aattagagtt gcagtaaatt tacagcactg cacgaataat aaggcattga agtttttcag 3480

tagaaattgt cctacagatg ctttatcgac ttgctattgg aagaatagat cttcttaaat 3540

gtgcagtgtt gagtcacttc gttatagtgg tagagttggg attagggctt caattttact 3600

tcttaaatat cattctatgt ttgatatgcc cagactgcat acaatttaaa gcaagagtac 3660

aactactatc gtaatggtaa tgtgaagatg ctattacaaa ggatctcctc ccaacccctc 3720

gggaatttgg tgtctttcaa attatatctt gaccttgaca tttgaatatc cagccattat 3780

tagatttctt aatggtgtga agtcccattt tcaataactt attggtgctg aaattgttca 3840

ctagctgtgg tctgacctag ttaatttaca agtacagatt gcataggacc cactagagaa 3900

gcatttatag tttgatggta agtagattag gcagaacgcc atctaaaata ttcttagaaa 3960

ataatgttga tgtattttcc atacctcatc agtttcactc aaccaataaa gtttttaaaa 4020

ttgtaacaaa gctcttagga tttacacatt tatatttaaa cattgataca tgaatattga 4080

ctgactgttg ataaagtcag agacaacttt tcctgagatc tcaccatgga aatctgtaca 4140

cccccttgtc tttcctaaaa gctgaaagtg gctgactaaa atgcaaagca gctgttgatg 4200

ttttgaagat agtgataaac actgttcttt gttagttttg ggcacagcat gctaaactat 4260

aacttgtatt gttccaatat gtaacacaga gggccaggtc atgaataatg acattacaat 4320

gggctgttgc actgttaata tttttccttt ggaatgtgaa ggtctgaatg agggttttga 4380

ttttgaatgt ttcagtgttt ttgagaagcc ttgcttacat tttatggtgt agtcattgga 4440

aatggaaaaa tggcattata tatatattat atatatataa atatatatat tatacatact 4500

ctccttactt tatttcagtt accatcccca tagaatttga caagaattgc tatgactgaa 4560

agggttttga gtcctaattc aaactttctt tatgacagta ttcacgatta gcctgaagtg 4620

cattctgtag gtgatctctc ccgtgtttct ggaatgcttt cttagactct tggatgtgca 4680

gcagcttatg tgtctgaaat gacttgaagg catcaccttt aagaaggctt acagttgggc 4740
cccgtacatc ccaagtcctc tgtaattcct cttggacatt tttgccataa ttgtaaaagg 4800
gtagttgaat taaatagcgt caccattctt tgctgtggca caggttataa acttaagtgg 4860
agtttaccgg cagcatcaaa tgtttcagct ttaaaaataa aagtaggtta caagttacat 4920
gtttagtttt agaaaatttg tgcaatatgt tcataacgat ggctgtggtt gccacaaagt 4980
gcctcgttta cctttaaata ctgttaatgt gtcgtgcatg cagacggaag gggtggatct 5040
gtgcactaaa cgggggcttt ttactctagt attcggcaga gttgccttct acctgccagc 5100
tcaaaagttc gatctgtttt catatagaat atatatacta aaaccatcca gtctgtaaaa 5160
cagccttacc ccgattcagc ctcttcagat actcttgtgc tgtgcagcag tggctctgtg 5220
tgtaaatgct atgcactgag gatacacaaa tatgacgtgt acaggataat gcctcatacc 5280
aatcagatgt ccatttgtta ctgtgtttgt taacaaccct ttatctctta gtgttataaa 5340
ctccacttaa aactgattaa agtctcattc ttgtcattgt gtgggtgttt tattaaatga 5400
gagtatttat aattcaaatt gcttaaatcc attaaaatgt tcagtaatgg gcagccacat 5460
atgattacaa agttcctgtg catttttcta tttttccccc tccttgctat ccttccaagc 5520
aaagcatctt tctgtcatct tggtagacac atacctgtct actcatggtt aagaagagca 5580
ctttaagcct tagtcatcac ttaataagtt attccaggca cagtaaaaag ttcaaggttc 5640
ttggaaaacg gtgcttattt ctcttcttat aagccagatg tctgaagata gccctaaccc 5700
caagaacggg cttgatgtct caggtctgtt ctgtggcttt ctgttttttt taacactgca 5760
gttggccatc agcacatggg aggtttcatc gggacttgtc cagagtagta ggctcaaata 5820
tactatctcc tttctaatat tcttaaaggc taaggagtcc tttcaatata acagtaagat 5880
aacttgtgat gttttagaag taagcagacc attaatgtca atgtggagtc ttaatgttac 5940
atgaagttga tagtttctct gtgacccatt taaaaataca aaccgagtag catgcaatta 6000
tgtaaagaaa tatgaagatt atatgtagtc acacattttc tttagaattc ttagtttggt 6060
gaaaacttga atataaaggt attttgattt atatgacatt ttgatgatat ttgaaaaaaa 6120
ggaatttcct gacattttgc ttttagatca tgtcccccat tgtgctgtaa tttaagccaa 6180
cttggttcag tgaatgccat caccatttcc attgagaatt taaaactcac cagtgtttaa 6240
catgcaggct tctgagggct cccggagaat cagaccttaa gcccagttga tttacttcta 6300
acgtgaaact tcgagttcct gtatactttg ctagataatt tgtggtacat ctaaagctta 6360
gtcttaagtg gcttgtgtgt ggattttatt caacattctt gttgctaggg tagagagaaa 6420
tgttgctgag tagaaacaag agtacccagt tcaatgtggt acagagagca gtccctaaaa 6480

tctgtacaca gtgtaatgga ccactttagg agtcaagagg ctgatttttc ctatgaaatt 6540

acattgcaac aggaagcctt ctagtatagt tccttttact gttagaatat gtttttatgc 6600

atacgctata gctgctttcc catcttccaa caacaggtat caggatgtaa gcaagcttta 6660

aacagtgtga agatggcagg atagtgtcat cggtaacagt cctctgactc taaatgtagt 6720

tgctctgtaa cactttgtga atataacatc acaattctca tgtccttggg gggggggggc 6780

atacccagta ttagtatgtt ttagtgacta agcaatcatt tttctgttta ctcatgtaca 6840

ttttctcttt aaaactaaaa cctgtactgt gtatgtctcc aaagcctttt agcttagttt 6900

ttaggaaatg aacactgaat ggatcacttt ttagtgtagc aggtatggga tatgtgcatt 6960

atagagagac cttgtcagct ctctgggcct atttgaatgt ttattgttgg tgtgaggatg 7020

gtaggggaat cagtaaatac aagttacgtt ggtttagcag agcaagctca gtgtgggtat 7080

ttctctttga agcgtggtgc gtgacgcact gtgagtagag aatttggtca ccctttgagt 7140

cctcttgcat tttgcaaact tgctcagcaa atgcgtacct accttgcccc ctaggtaaaa 7200

gcaggaacta ctactgattt atctgtcact cagctgtctt tatatgtgtg cttctgtgac 7260

ttgtatcaca caagaatctt aaagatttca caaattgtta ccttttagct ctgaatgttg 7320

agtattctgg tgggctaaca acaagacaaa ctcttgacag tcatttgaga attttcatga 7380

aacatttagc tgaaaacatt ttataattta tgaaaaaaat gtgttacctt aaacttttac 7440

atatgtggga gacattaact gccatatttg agcatactga attttaaatt taaaataaag 7500

ctgcatattt ttaaatgaaa tgtttaacaa ggattcatat tttttgtttt ttaagattaa 7560

aaataattta tgtcttctca tgtggaacct catctgtcac aatggttaga ttatacagaa 7620

tggagcaagg cttgtagtgg tttagcttac agtaaaattc ttaatgttta gatgtgttta 7680

cttactggct gttatgtata cttttgagat tttccacctg ttctgtgtag ttttctaaat 7740

gatactccta cttaaaaaca gcattttagt atctattttc tgtctccatt aaatggtcct 7800

cattttctat tgagtttgga agtgtgcaca ttgtgtgtgt gtgtgtgtgt gtgtgtgtgc 7860

acacgtgtgc gcgcccgtgc gtgtgtctat ttgtggagtt tgtatgggag aattagtttt 7920

gaaagtgcta gaatagagat gaaatttggt tcaagtaaaa ttttcccact gggattttac 7980

agtttattgt aataaaatgt taattttgga tgaccttgaa tattaatgaa tttgttagcc 8040

tcttgatgtg tgcattaatg agatatatca aagttgtata ttaaaccaaa gttggagttg 8100

tggaagtgtt tttatgaagt tccgtttggc taccaatgga cataagacta gaaatacctt 8160

cctgtggaga atatttttcc tttaaacaat taaaaaggtt cattattttt gaaaaaaaaa 8220

aaaaaaaaa 8229

<210> 4
<211> 1212
<212> DNA
<213> Rattus norvegicus

<400> 4
atgacagcca tcatcaaaga gatcgttagc agaaacaaaa ggagatatca agaggatgga 60

ttcgacttag acttgaccta tatttatcca aatattattg ctatgggatt tcctgcagaa 120

agacttgaag gtgtatacag gaacaatatt gatgatgtag taaggttttt ggattcaaag 180

cataaaaacc attacaagat atacaatcta tgtgctgaga gacattatga caccgccaaa 240

tttaactgca gagttgcaca gtatcctttt gaagaccata acccaccaca gctagaactt 300

atcaaaccct tttgtgaaga tcttgaccaa tggctaagtg aagacgacaa tcatgttgca 360

gcaattcact gtaaagctgg gaaaggacgg actggtgtaa tgatttgtgc atatttattg 420

catcggggca agtttttaaa ggcacaagag gccctggatt tttatgggga agtaaggacc 480

agagataaaa agggagtaac tattcccagt cagaggcgct atgtatatta ttatagctac 540

ctgttaaaga atcacctgga ttacagacca gtggcactgt tgtttcacaa gatgatgttt 600

gaaactattc caatgttcag tggcggaact tgcaatcccc agtttgtggt ctgccagcta 660

aaggtgaaga tctactcctc caactcagga cccacgcggc gggaggacaa gctcatgtac 720

tttgagttcc ctcagccatt gcctgtgtgt ggtgacatca aagtagagtt cttccacaaa 780

cagaacaaga tgctcaaaaa ggacaaaatg tttcactttt gggtaaatac gttcttcata 840

ccaggaccag aggaaacctc agaaaaagtg gaaaatggaa gtctttgtga tcaggaaatc 900

gatagcattt gtagtataga gcgtgcggat aatgacaagg agtatcttgt gctcaccctg 960

acaaaaaatg atcttgacaa agcaaacaaa gacaaggcca accgatactt ctctccaaat 1020

tttaaggtga agttatactt cacaaaaaca gtagaggagc catcaaatcc agaggctagc 1080

agttcaactt ctgtgactcc agacgttagt gacaatgaac ctgatcatta tagatattct 1140

gacaccactg actctgatcc agagaatgaa ccttttgatg aagatcagca ttcacaaatt 1200

acaaaagtct ga 1212

<210> 5
<211> 1396
<212> DNA
<213> Canis familiaris

<400> 5
ccgcccgccg ccaggcccgg ggccgcctgc agcctgcgga ggaggccgcg ccgcccgccg 60

ctcctgccgt ctctctcctc cttcctctcc agccaccggc tcccagacat gacagccatc 120

atcaaggaga tcgtcagcag aaacaaaagg cgctaccagg aggatgggtt cgacttggac 180

ttgacctata tttatcccaa cattattgct atggggtttc ctgcagaaag acttgaaggc 240

gtatacagga acaatattga tgatgtagta aggtttttgg attcaaagca taaaaaccat 300

tacaagatat acaatctgtg tgctgaaaga cattatgata ccgccaaatt taactgcaga 360

gttgcacagt atccttttga agaccataat ccaccacagc tagaacttat caaacccttt 420

tgtgaagatc ttgaccaatg gctaagtgaa gatgacaatc atgttgcagc aattcactgt 480

aaagctggaa agggacgaac tggtgtaatg atttgtgcat atttattaca tcggggcaaa 540

tttctaaagg cacaagaggc cctagatttc tatggggaag taaggaccag agacaaaaag 600

ggagtaacta ttcccagtca gaggcgctat gtgtattatt atagctacct gttaaagaat 660

catctggatt atagaccagt ggcactgttg tttcacaaga tgatgtttga aactattcca 720

atgttcagtg gcggaacttg caatcctcag tttgtggtct gccagctaaa ggtgaagatc 780

tattcctcca attcaggacc cacacgacgg gaagacaagt tcatgtactt tgagttccct 840

cagccattgc ctgtgtgcgg tgacatcaaa gtagagttct tccacaaaca gaacaagatg 900

ctaaaaaagg acaaaatgtt tcacttttgg gtaaacacat tcttcatacc aggaccagag 960

gaaacctcag aaaaagtaga aaatggaagt ctatgtgatc aagaaattga tagtatttgc 1020

agtatagaac gtgcagataa tgacaaggaa tatctagtac tcactttaac aaaaaatgat 1080

ctcgacaaag caaataaaga caaggccaac cgatattttt ctccaaattt taaggtgaag 1140

ctgtacttca caaaaactgt agaggagcca tcaaacccgg aggctagcag ttcaacttct 1200

gtgacgccag atgttagtga caatgaacct gatcattata gatattctga caccactgac 1260

tctgacccag agaatgaacc ctttgatgaa gatcagcaca cacaaatcac aaaagtctga 1320

atttttttta atcaagaggg ataaaacacc atgaaaacaa acttgaataa actgaaattg 1380

gacctttttt ttttaa 1396

<210> 6
<211> 5616
<212> DNA
<213> Homo sapiens

<400> 6
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380

gactccttca cacatactcc tcctctggat ccacaggaac tggatattct gaaaaccgta 1440

aaggaaatca cagggttttt gctgattcag gcttggcctg aaaacaggac ggacctccat 1500

gcctttgaga acctagaaat catacgcggc aggaccaagc aacatggtca gttttctctt 1560

gcagtcgtca gcctgaacat aacatccttg ggattacgct ccctcaagga gataagtgat 1620

ggagatgtga taatttcagg aaacaaaaat ttgtgctatg caaatacaat aaactggaaa 1680

aaactgtttg ggacctccgg tcagaaaacc aaaattataa gcaacagagg tgaaaacagc 1740

tgcaaggcca caggccaggt ctgccatgcc ttgtgctccc ccgagggctg ctggggcccg 1800

gagcccaggg actgcgtctc ttgccggaat gtcagccgag gcagggaatg cgtggacaag 1860

tgcaaccttc tggagggtga gccaagggag tttgtggaga actctgagtg catacagtgc 1920

cacccagagt gcctgcctca ggccatgaac atcacctgca caggacgggg accagacaac 1980

tgtatccagt gtgcccacta cattgacggc ccccactgcg tcaagacctg cccggcagga 2040

gtcatgggag aaaacaacac cctggtctgg aagtacgcag acgccggcca tgtgtgccac 2100

ctgtgccatc caaactgcac ctacggatgc actgggccag gtcttgaagg ctgtccaacg 2160

aatgggccta agatcccgtc catcgccact gggatggtgg gggccctcct cttgctgctg 2220

gtggtggccc tggggatcgg cctcttcatg cgaaggcgcc acatcgttcg gaagcgcacg 2280

ctgcggaggc tgctgcagga gagggagctt gtggagcctc ttacacccag tggagaagct 2340

cccaaccaag ctctcttgag gatcttgaag gaaactgaat tcaaaaagat caaagtgctg 2400

ggctccggtg cgttcggcac ggtgtataag ggactctgga tcccagaagg tgagaaagtt 2460

aaaattcccg tcgctatcaa ggaattaaga gaagcaacat ctccgaaagc caacaaggaa 2520

atcctcgatg aagcctacgt gatggccagc gtggacaacc cccacgtgtg ccgcctgctg 2580

ggcatctgcc tcacctccac cgtgcagctc atcacgcagc tcatgccctt cggctgcctc 2640

ctggactatg tccgggaaca caaagacaat attggctccc agtacctgct caactggtgt 2700

gtgcagatcg caaagggcat gaactacttg gaggaccgtc gcttggtgca ccgcgacctg 2760

gcagccagga acgtactggt gaaaacaccg cagcatgtca agatcacaga ttttgggctg 2820

gccaaactgc tgggtgcgga agagaaagaa taccatgcag aaggaggcaa agtgcctatc 2880

aagtggatgg cattggaatc aattttacac agaatctata cccaccagag tgatgtctgg 2940

agctacgggg tgaccgtttg ggagttgatg acctttggat ccaagccata tgacggaatc 3000

cctgccagcg agatctcctc catcctggag aaaggagaac gcctccctca gccacccata 3060

tgtaccatcg atgtctacat gatcatggtc aagtgctgga tgatagacgc agatagtcgc 3120

ccaaagttcc gtgagttgat catcgaattc tccaaaatgg cccgagaccc ccagcgctac 3180

cttgtcattc agggggatga aagaatgcat ttgccaagtc ctacagactc caacttctac 3240

cgtgccctga tggatgaaga agacatggac gacgtggtgg atgccgacga gtacctcatc 3300

ccacagcagg gcttcttcag cagcccctcc acgtcacgga ctcccctcct gagctctctg 3360

agtgcaacca gcaacaattc caccgtggct tgcattgata gaaatgggct gcaaagctgt 3420

cccatcaagg aagacagctt cttgcagcga tacagctcag accccacagg cgccttgact 3480

gaggacagca tagacgacac cttcctccca gtgcctgaat acataaacca gtccgttccc 3540

aaaaggcccg ctggctctgt gcagaatcct gtctatcaca atcagcctct gaaccccgcg 3600

cccagcagag acccacacta ccaggacccc cacagcactg cagtgggcaa ccccgagtat 3660

ctcaacactg tccagcccac ctgtgtcaac agcacattcg acagccctgc ccactgggcc 3720

cagaaaggca gccaccaaat tagcctggac aaccctgact accagcagga cttctttccc 3780

aaggaagcca agccaaatgg catctttaag ggctccacag ctgaaaatgc agaataccta 3840

agggtcgcgc cacaaagcag tgaatttatt ggagcatgac cacggaggat agtatgagcc 3900

ctaaaaatcc agactctttc gatacccagg accaagccac agcaggtcct ccatcccaac 3960

agccatgccc gcattagctc ttagacccac agactggttt tgcaacgttt acaccgacta 4020

gccaggaagt acttccacct cgggcacatt ttgggaagtt gcattccttt gtcttcaaac 4080

tgtgaagcat ttacagaaac gcatccagca agaatattgt ccctttgagc agaaatttat 4140

ctttcaaaga ggtatatttg aaaaaaaaaa aaagtatatg tgaggatttt tattgattgg 4200

ggatcttgga gtttttcatt gtcgctattg atttttactt caatgggctc ttccaacaag 4260

gaagaagctt gctggtagca cttgctaccc tgagttcatc caggcccaac tgtgagcaag 4320

gagcacaagc cacaagtctt ccagaggatg cttgattcca gtggttctgc ttcaaggctt 4380

ccactgcaaa acactaaaga tccaagaagg ccttcatggc cccagcaggc cggatcggta 4440

ctgtatcaag tcatggcagg tacagtagga taagccactc tgtcccttcc tgggcaaaga 4500

agaaacggag ggatggaatt tcttccttag acttactttt gtaaaaatgt ccccacggta 4560

cttactcccc actgatggac cagtggtttc cagtcatgag cgttagactg acttgtttgt 4620

cttccattcc attgttttga aactcagtat gctgcccctg tcttgctgtc atgaaatcag 4680

caagagagga tgacacatca aataataact cggattccag cccacattgg attcatcagc 4740

atttggacca atagcccaca gctgagaatg tggaatacct aaggatagca ccgcttttgt 4800

tctcgcaaaa acgtatctcc taatttgagg ctcagatgaa atgcatcagg tcctttgggg 4860

catagatcag aagactacaa aaatgaagct gctctgaaat ctcctttagc catcacccca 4920

accccccaaa attagtttgt gttacttatg gaagatagtt ttctcctttt acttcacttc 4980

aaaagctttt tactcaaaga gtatatgttc cctccaggtc agctgccccc aaaccccctc 5040

cttacgcttt gtcacacaaa aagtgtctct gccttgagtc atctattcaa gcacttacag 5100

ctctggccac aacagggcat tttacaggtg cgaatgacag tagcattatg agtagtgtgg 5160

aattcaggta gtaaatatga aactagggtt tgaaattgat aatgctttca caacatttgc 5220

agatgtttta gaaggaaaaa agttccttcc taaaataatt tctctacaat tggaagattg 5280

gaagattcag ctagttagga gcccaccttt tttcctaatc tgtgtgtgcc ctgtaacctg 5340

actggttaac agcagtcctt tgtaaacagt gttttaaact ctcctagtca atatccaccc 5400

catccaattt atcaaggaag aaatggttca gaaaatattt tcagcctaca gttatgttca 5460

gtcacacaca catacaaaat gttccttttg cttttaaagt aatttttgac tcccagatca 5520

gtcagagccc ctacagcatt gttaagaaag tatttgattt ttgtctcaat gaaaataaaa 5580

ctatattcat ttccactcta aaaaaaaaaa aaaaaa 5616

<210> 7
<211> 2239
<212> DNA
<213> Homo sapiens

<400> 7
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380

gactccttca cacatactcc tcctctggat ccacaggaac tggatattct gaaaaccgta 1440

aaggaaatca cagggttttt gctgattcag gcttggcctg aaaacaggac ggacctccat 1500

gcctttgaga acctagaaat catacgcggc aggaccaagc aacatggtca gttttctctt 1560

gcagtcgtca gcctgaacat aacatccttg ggattacgct ccctcaagga gataagtgat 1620

ggagatgtga taatttcagg aaacaaaaat ttgtgctatg caaatacaat aaactggaaa 1680

aaactgtttg ggacctccgg tcagaaaacc aaaattataa gcaacagagg tgaaaacagc 1740

tgcaaggcca caggccaggt ctgccatgcc ttgtgctccc ccgagggctg ctggggcccg 1800

gagcccaggg actgcgtctc ttgccggaat gtcagccgag gcagggaatg cgtggacaag 1860

tgcaaccttc tggagggtga gccaagggag tttgtggaga actctgagtg catacagtgc 1920

cacccagagt gcctgcctca ggccatgaac atcacctgca caggacgggg accagacaac 1980

tgtatccagt gtgcccacta cattgacggc ccccactgcg tcaagacctg cccggcagga 2040

gtcatgggag aaaacaacac cctggtctgg aagtacgcag acgccggcca tgtgtgccac 2100

ctgtgccatc caaactgcac ctacgggtcc taataaatct tcactgtctg actttagtct 2160

cccactaaaa ctgcatttcc tttctacaat ttcaatttct ccctttgctt caaataaagt 2220

cctgacacta ttcatttga 2239

<210> 8
<211> 1595
<212> DNA
<213> Homo sapiens

<400> 8
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380

gactccttca cacatactcc tcctctggat ccacaggaac tggatattct gaaaaccgta 1440

aaggaaatca caggtttgag ctgaattatc acatgaatat aaatggaaaa tcagtgtttt 1500

agagagagaa cttttcgaca tatttcctgt tcccttggaa taaaaacatt tcttctgaaa 1560

ttttaccgtt aaaaaaaaaa aaaaaaaaaa aaaaa 1595

<210> 9
<211> 2865
<212> DNA
<213> Homo sapiens

<400> 9
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380

gactccttca cacatactcc tcctctggat ccacaggaac tggatattct gaaaaccgta 1440

aaggaaatca cagggttttt gctgattcag gcttggcctg aaaacaggac ggacctccat 1500

gcctttgaga acctagaaat catacgcggc aggaccaagc aacatggtca gttttctctt 1560

gcagtcgtca gcctgaacat aacatccttg ggattacgct ccctcaagga gataagtgat 1620

ggagatgtga taatttcagg aaacaaaaat ttgtgctatg caaatacaat aaactggaaa 1680

aaactgtttg ggacctccgg tcagaaaacc aaaattataa gcaacagagg tgaaaacagc 1740

tgcaaggcca caggccaggt ctgccatgcc ttgtgctccc ccgagggctg ctggggcccg 1800

gagcccaggg actgcgtctc ttgccggaat gtcagccgag gcagggaatg cgtggacaag 1860

tgcaaccttc tggagggtga gccaagggag tttgtggaga actctgagtg catacagtgc 1920

cacccagagt gcctgcctca ggccatgaac atcacctgca caggacgggg accagacaac 1980

tgtatccagt gtgcccacta cattgacggc ccccactgcg tcaagacctg cccggcagga 2040

gtcatgggag aaaacaacac cctggtctgg aagtacgcag acgccggcca tgtgtgccac 2100

ctgtgccatc caaactgcac ctacggccca ggaaatgaga gtctcaaagc catgttattc 2160

tgccttttta aactatcatc ctgtaatcaa agtaatgatg gcagcgtgtc ccaccagagc 2220

gggagcccag ctgctcagga gtcatgctta ggatggatcc cttctcttct gccgtcagag 2280

tttcagctgg gttggggtgg atgcagccac ctccatgcct ggccttctgc atctgtgatc 2340

atcacggcct cctcctgcca ctgagcctca tgccttcacg tgtctgttcc ccccgctttt 2400

cctttctgcc acccctgcac gtgggccgcc aggttcccaa gagtatccta cccatttcct 2460

tccttccact ccctttgcca gtgcctctca ccccaactag tagctaacca tcacccccag 2520

gactgacctc ttcctcctcg ctgccagatg attgttcaaa gcacagaatt tgtcagaaac 2580

ctgcagggac tccatgctgc cagccttctc cgtaattagc atggccccag tccatgcttc 2640

tagccttggt tccttctgcc cctctgtttg aaattctaga gccagctgtg ggacaattat 2700

ctgtgtcaaa agccagatgt gaaaacatct caataacaaa ctggctgctt tgttcaatgc 2760

tagaacaacg cctgtcacag agtagaaact caaaaatatt tgctgagtga atgaacaaat 2820

gaataaatgc ataataaata attaaccacc aatccaacat ccaga 2865

<210> 10
<211> 5983
<212> DNA
<213> Mus musculus

<400> 10
ctcccccagt cccgacccga gctaactaga cgtctgggca gccccagcgc aacgcgcagc 60

agcctccctc ctcttcttcc cgcactgtgc gctcctcctg ggctagggcg tctggatcga 120

gtcccggagg ctaccgcctc ccagacagac gacaggtcac ctggacgcga gcctgtgtcc 180

gggtctcgtc gttgccggcg cagtcactgg gcacaaccgt gggactccgt ctgtctcgga 240

ttaatcccgg agagccagag ccaacctctc ccggtcagag atgcgaccct cagggaccgc 300

gagaaccaca ctgctggtgt tgctgaccgc gctctgcgcc gcaggtgggg cgttggagga 360

aaagaaagtc tgccaaggca caagtaacag gctcacccaa ctgggcactt ttgaagacca 420

ctttctgagc ctgcagagga tgtacaacaa ctgtgaagtg gtccttggga acttggaaat 480

tacctatgtg caaaggaatt acgacctttc cttcttaaag accatccagg aggtggccgg 540

ctatgtcctc attgccctca acaccgtgga gagaatccct ttggagaacc tgcagatcat 600

caggggaaat gctctttatg aaaacaccta tgccttagcc atcctgtcca actatgggac 660

aaacagaact gggcttaggg aactgcccat gcggaactta caggaaatcc tgattggtgc 720

tgtgcgattc agcaacaacc ccatcctctg caatatggat actatccagt ggagggacat 780

cgtccaaaac gtctttatga gcaacatgtc aatggactta cagagccatc cgagcagttg 840

ccccaaatgt gatccaagct gtcccaatgg aagctgctgg ggaggaggag aggagaactg 900

ccagaaattg accaaaatca tctgtgccca gcaatgttcc catcgctgtc gtggcaggtc 960

ccccagtgac tgctgccaca accaatgtgc tgcggggtgt acagggcccc gagagagtga 1020

ctgtctggtc tgccaaaagt tccaagatga ggccacatgc aaagacacct gcccaccact 1080

catgctgtac aaccccacca cctatcagat ggatgtcaac cctgaaggga agtacagctt 1140

tggtgccacc tgtgtgaaga agtgcccccg aaactacgtg gtgacagatc atggctcatg 1200

tgtccgagcc tgtgggcctg actactacga agtggaagaa gatggcatcc gcaagtgtaa 1260

aaaatgtgat gggccctgtc gcaaagtttg taatggcata ggcattggtg aatttaaaga 1320

cacactctcc ataaatgcta caaacatcaa acacttcaaa tactgcactg ccatcagcgg 1380

ggaccttcac atcctgccag tggcctttaa gggggattct ttcacgcgca ctcctcctct 1440

agacccacga gaactagaaa ttctaaaaac cgtaaaggaa ataacaggct ttttgctgat 1500

tcaggcttgg cctgataact ggactgacct ccatgctttc gagaacctag aaataatacg 1560

tggcagaaca aagcaacatg gtcagttttc tttggcggtc gttggcctga acatcacatc 1620

actggggctg cgttccctca aggagatcag tgatggggat gtgatcattt ctggaaaccg 1680

aaatttgtgc tacgcaaaca caataaactg gaaaaaactc ttcgggacac ccaatcagaa 1740

aaccaaaatc atgaacaaca gagctgagaa agactgcaag gccgtgaacc acgtctgcaa 1800

tcctttatgc tcctcggaag gctgctgggg ccctgagccc agggactgtg tctcctgcca 1860

gaatgtgagc agaggcaggg agtgcgtgga gaaatgcaac atcctggagg gggaaccaag 1920

ggagtttgtg gaaaattctg aatgcatcca gtgccatcca gaatgtctgc cccaggccat 1980

gaacatcacc tgtacaggca ggggaccaga caactgcatc cagtgtgccc actacattga 2040

tggcccacac tgtgtcaaga cctgcccagc tggcatcatg ggagagaaca acactctggt 2100

ctggaagtat gcagatgcca ataatgtctg ccacctatgc cacgccaact gtacctatgg 2160

atgtgctggg ccaggtcttc aaggatgtga agtgtggcca tctgggccaa agataccatc 2220

tattgccact gggattgtgg gtggcctcct cttcatagtg gtggtggccc ttgggattgg 2280

cctattcatg cgaagacgtc acattgttcg aaagcgtaca ctacgccgcc tgcttcaaga 2340

gagagagctc gtggaacctc tcacacccag cggagaagct ccaaaccaag cccacttgag 2400

gatattaaag gaaacagaat tcaaaaagat caaagttctg ggttcgggag catttggcac 2460

agtgtataag ggtctctgga tcccagaagg tgagaaagta aaaatcccgg tggccatcaa 2520

ggagttaaga gaagccacat ctccaaaagc caacaaagaa atccttgacg aagcctatgt 2580

gatggctagt gtggacaacc ctcatgtatg ccgcctcctg ggcatctgtc tgacctccac 2640

tgtccagctc attacacagc tcatgcccta cggttgcctc ctggactacg tccgagaaca 2700

caaggacaac attggctccc agtacctcct caactggtgt gtgcagattg caaagggcat 2760

gaactacctg gaagatcggc gtttggtgca ccgtgacttg gcagccagga atgtactggt 2820

gaagacacca cagcatgtca agatcacaga ttttgggctg gccaaactgc ttggtgctga 2880

agagaaagaa tatcatgccg aggggggcaa agtgcctatc aagtggatgg ctttggaatc 2940

aattttacac cgaatttata cacaccaaag tgatgtctgg agctatggtg tcactgtgtg 3000

ggaactgatg acctttgggt ccaagcctta tgatggaatc ccagcaagtg acatctcatc 3060

catcctagag aaaggagagc gccttccaca gccacctatc tgcaccatcg atgtctacat 3120

gatcatggtc aagtgctgga tgatagatgc tgatagccgc ccaaagttcc gagagttgat 3180

tcttgaattc tccaaaatgg cccgagaccc acagcgctac cttgttatcc agggggatga 3240

aagaatgcat ttgccaagcc ctacagactc caacttttac cgagccctga tggatgaaga 3300

ggacatggag gatgtagttg atgctgatga gtatcttatc ccacagcaag gcttcttcaa 3360

cagcccgtcc acgtcgagga ctcccctctt gagttctctg agtgcaacta gcaacaattc 3420

cactgtggct tgcattaata gaaatgggag ctgccgtgtc aaagaagacg ccttcttgca 3480

gcggtacagc tccgacccca caggtgctgt aacagaggac aacatagatg acgcattcct 3540

ccctgtacct gaatatgtaa accaatctgt tcccaagagg ccagcaggct ctgtgcagaa 3600

ccctgtctat cacaatcagc ccctgcatcc agctcctgga agagacctgc attatcaaaa 3660

tccccacagc aatgcagtgg gcaaccctga gtatctcaac actgcccagc ctacctgtct 3720

cagtagtggg tttaacagcc ctgcactctg gatccagaaa ggcagtcacc aaatgagcct 3780

agacaaccct gactaccagc aggacttctt ccccaaggaa accaagccaa atggcatatt 3840

taagggcccc acagctgaaa atgcagagta cctacgggtg gcacctccaa gcagtgagtt 3900

tattggagca tgacaagaag gggcatcata ccagctataa aatgtctgga ctttctagaa 3960

tcccaggacc aactatggca gcacctccac ttctggtagc catgcccacg ctgtgtcaaa 4020

tgtcactcag actggcttta aagcataact ctgatgggct ttgtcactga gccaagaagt 4080

gggcctctct cctgatgcac tttgggaagt tgaaggtaca tcaattgatc ttcgaactgt 4140

gaagattcca caaaaaaggt atccatcgag aacattgtcc attggaacag aagtttgcct 4200

catggtgagg tacatatggg aaaaaaacag acatatggag cttatattta gggaactttg 4260

ggattcttgt ctttattgat ttgattgatg cactcttgta gtctggtaca cagagttgcc 4320

tggagccaac tgaccagaca gttggttcca ccagctctgc atcaagacac ttccgtggca 4380

agacaactaa atgtataaga agtccatgga tgccctgagc aggccacact tgtacagcat 4440

taaaccatgg cagatacaat aggataagcc actttgttac ttactggggc tgggagaaga 4500

ggaatgacgg ggtagaattt tccctcagac gtacttttta tataaatatg tccctggcac 4560

ctaacacgcg ctagtttacc agtgttttct attagacttc cttctatgtt ttctgtttca 4620

ttgttttgag ttgtaaatat gtgttcctgt cttcatttca tgaagtaaac aaacaaacaa 4680

aaaacccagt attaagtatt atcaaagaac aaccatgatt ccacattcga acccattcaa 4740

accatcagta ttgtgaccaa aagcctttaa ctaagaagga gtaaccatgc aaaaatccat 4800

agaggaattt aacccaaaat tttagtctca gcattgtgtc tgctgaggtg tgtatatgag 4860

actacgaaag tgaactactc ttcaaatcca ctttgccttc actcctctat accctaaatc 4920

tagtgtaaac cacacatgga ggataacttt tttttttaat tttaaaagtg tttattagat 4980

atgtttttct tcctggtaaa ctgcagccaa acatcagtta agagccattt ttgataaaca 5040

ctatcacaat gatctcggga tccatccttt ccgatttacc aagtgatgga tagacgtgaa 5100

ctcataaaca ctacccataa gacaaaacaa tgagtgccag acaagacatc agccaggcac 5160

cagagcacag agcaggactg ggcaatctgt tggagatatc tagaaagttc acaaaggaaa 5220

caagattgtc cactaccttg tgagatctag cagtcataaa taccagggaa atggaaagtg 5280

tgtttcctta cagcaccagg tcttcgatct tcctaatgct gtgacccttt aatacagttt 5340

gccatgttgt ggtgaccccc aaccataaaa ttatttttgt tgctacttca taactgtaaa 5400

tttgctactc ttacagacca caatgtaaat atctgatatg ctatctgata tgcaggctat 5460

ctgacagagg tcgcaacccg caggttgaga gccactgcct tcaaggcttt aatcaagaga 5520

gtagtgagct gagggcttta ctggtaagtc aggggcaagt ccaactcaat catcctcaca 5580

tactggctgc tccctcaggc ctgagaatga ggcttgcagc atcctctggt ttcctaaccg 5640

ttatccatcc ctgactctca tctctgaaaa tagatgtcat ccatgaaatt aaggagtgag 5700

aatattaagc agcatttata gagctcaaaa ttccatgtca tcaccaggaa gtgccatgtt 5760

gatcacagag aacacagagg agacatatag acaggttttt gctcaaaatt gggatataga 5820

atgagcctgt caggtaccta tcaggagcgg taatccgtga gagagaaccg ttgcaagcca 5880

ctctaactgt agcaatgaaa ccctagtatt tttgtacttt gaaatacttt cttataacaa 5940

aataaagtag caaaaaaact gttcaaaaaa aaaaaaaaaa aaa 5983

<210> 11
<211> 2678
<212> DNA
<213> Mus musculus

<400> 11
ctcccccagt cccgacccga gctaactaga cgtctgggca gccccagcgc aacgcgcagc 60

agcctccctc ctcttcttcc cgcactgtgc gctcctcctg ggctagggcg tctggatcga 120

gtcccggagg ctaccgcctc ccagacagac gacaggtcac ctggacgcga gcctgtgtcc 180

gggtctcgtc gttgccggcg cagtcactgg gcacaaccgt gggactccgt ctgtctcgga 240

ttaatcccgg agagccagag ccaacctctc ccggtcagag atgcgaccct cagggaccgc 300

gagaaccaca ctgctggtgt tgctgaccgc gctctgcgcc gcaggtgggg cgttggagga 360

aaagaaagtc tgccaaggca caagtaacag gctcacccaa ctgggcactt ttgaagacca 420

ctttctgagc ctgcagagga tgtacaacaa ctgtgaagtg gtccttggga acttggaaat 480

tacctatgtg caaaggaatt acgacctttc cttcttaaag accatccagg aggtggccgg 540

ctatgtcctc attgccctca acaccgtgga gagaatccct ttggagaacc tgcagatcat 600

caggggaaat gctctttatg aaaacaccta tgccttagcc atcctgtcca actatgggac 660

aaacagaact gggcttaggg aactgcccat gcggaactta caggaaatcc tgattggtgc 720

tgtgcgattc agcaacaacc ccatcctctg caatatggat actatccagt ggagggacat 780

cgtccaaaac gtctttatga gcaacatgtc aatggactta cagagccatc cgagcagttg 840

ccccaaatgt gatccaagct gtcccaatgg aagctgctgg ggaggaggag aggagaactg 900

ccagaaattg accaaaatca tctgtgccca gcaatgttcc catcgctgtc gtggcaggtc 960

ccccagtgac tgctgccaca accaatgtgc tgcggggtgt acagggcccc gagagagtga 1020

ctgtctggtc tgccaaaagt tccaagatga ggccacatgc aaagacacct gcccaccact 1080

catgctgtac aaccccacca cctatcagat ggatgtcaac cctgaaggga agtacagctt 1140

tggtgccacc tgtgtgaaga agtgcccccg aaactacgtg gtgacagatc atggctcatg 1200

tgtccgagcc tgtgggcctg actactacga agtggaagaa gatggcatcc gcaagtgtaa 1260

aaaatgtgat gggccctgtc gcaaagtttg taatggcata ggcattggtg aatttaaaga 1320

cacactctcc ataaatgcta caaacatcaa acacttcaaa tactgcactg ccatcagcgg 1380

ggaccttcac atcctgccag tggcctttaa gggggattct ttcacgcgca ctcctcctct 1440

agacccacga gaactagaaa ttctaaaaac cgtaaaggaa ataacaggct ttttgctgat 1500

tcaggcttgg cctgataact ggactgacct ccatgctttc gagaacctag aaataatacg 1560

tggcagaaca aagcaacatg gtcagttttc tttggcggtc gttggcctga acatcacatc 1620

actggggctg cgttccctca aggagatcag tgatggggat gtgatcattt ctggaaaccg 1680

aaatttgtgc tacgcaaaca caataaactg gaaaaaactc ttcgggacac ccaatcagaa 1740

aaccaaaatc atgaacaaca gagctgagaa agactgcaag gccgtgaacc acgtctgcaa 1800

tcctttatgc tcctcggaag gctgctgggg ccctgagccc agggactgtg tctcctgcca 1860

gaatgtgagc agaggcaggg agtgcgtgga gaaatgcaac atcctggagg gggaaccaag 1920

ggagtttgtg gaaaattctg aatgcatcca gtgccatcca gaatgtctgc cccaggccat 1980

gaacatcacc tgtacaggca ggggaccaga caactgcatc cagtgtgccc actacattga 2040

tggcccacac tgtgtcaaga cctgcccagc tggcatcatg ggagagaaca acactctggt 2100

ctggaagtat gcagatgcca ataatgtctg ccacctatgc cacgccaact gtacctatgg 2160

atgtgctggg ccaggtcttc aaggatgtga agtgtggcca tctgggtacg ttcaatggca 2220

gtggatctta aagacctttt ggatctaaga ccagaagcca tctctgactc ccctctcacc 2280

ttccagtttc ttccaaatcc tctgggccag ccagaggtct cagattctgc cctcttgccc 2340

tgtgcccacc ttgttgacca ctggacagca tatgtgatgg ctactgctag tgccagcttc 2400

acaagaggtt aacactacgg actagccatt cttcctatgt atctgtttct gcaaatacag 2460

ccgctttact taagtctcag cacttcttag tctcctcttt tcctctcagt agcccaaggg 2520

gtcatgtcac aaacatggtg tgaagggcta ctttgtcaaa tgaaaaggtc tatcttgggg 2580

ggcatttttt tcttttcttt ttttcttgaa acacattgcc cagcaaagcc aataaatttc 2640

tctcatcatt ttgtttctga taaattctta ctattgat 2678

<210> 12
<211> 4161
<212> DNA
<213> Rattus norvegicus

<400> 12
ggaccgccac caagacaggc gacgggtcac ctggacgcga gtctgagtcc gggtcccgtc 60

gtcgttgccg acgcagtcac cgggcacgac cgtgggactc agtctgactc ggattaatcc 120

cggagagcca gagccaacga ctgccgagcc gggatgcgac cctcagggac tgcgagaacc 180

aagctactgc tgctgctggc tgcgctctgc gccgcaggtg gggcgctgga ggaaaagaaa 240

gtttgccaag gcacaagtaa caggctcacc caactaggca cctttgaaga ccactttctg 300

agcctccaga ggatgttcaa caactgtgaa gtggtccttg gaaacttgga aatcacctat 360

gtgcaaagga attatgacct ttccttctta aagaccatcc aggaggtggc tggctatgtt 420

ctcattgccc tgaacaccgt ggagagaatc cctttggaga acctgcagat catcagggga 480

aatgctctct acgaaaacac ctacgcctta gccgtcctgt ccaactatgg aaccaacaaa 540

actgggctta gggaactgcc catgcggaac ttacaggaaa ttctgatcgg tgctgtgcga 600

tttagcaaca accccatcct ctgcaatatg gagaccatcc agtggaggga catcgtccaa 660

gatgtctttc tgagcaacat gtcaatggac gtacagcgcc acctgacggg ctgcccgaaa 720

tgtgatccga gctgtcccaa tggaagctgc tggggaagag gagaggagaa ctgccagaaa 780

ttgaccaaaa tcatctgcgc ccagcaatgt tcccggcgtt gtcgtggcag gtcccctagc 840

gactgctgcc acaaccagtg tgccgcaggg tgtacagggc ccagagagag tgactgtctg 900

gtctgccaca ggttccgaga tgaagccacg tgcaaagaca cctgcccacc actcatgctg 960

tacaacccca ccacgtacca gatggatgtc aaccctgagg ggaagtacag ctttggtgcc 1020

acctgtgtga agaaatgccc cagaaactac gtggtgacag atcacggctc gtgtgtccgg 1080

gcctgtgggc cagactacta tgaagtagaa gaagatggag tcagcaagtg taaaaaatgt 1140

gacgggccct gccgcaaagt ttgcaatggc ataggcattg gtgaatttaa agacacactc 1200

tccataaatg ctacaaacat caaacacttc aagtactgca ctgccatcag tggggacctc 1260

cacatcctgc cagtggcctt taagggggat tctttcaccc gcactcctcc tctagaccca 1320

cgggaactag aaattctcaa aactgtgaag gaaataacag ggtttttgct gattcaggct 1380

tggcctgaaa actggactga cctccatgct tttgagaacc tagaaataat tcgtggcaga 1440

acaaagcaac atggtcagtt ttctctggcg gttgtcggcc tgaacataac atcgctgggg 1500

ttgcgttccc tcaaggagat cagtgatggg gatgtgatta tttctgggaa ccgaaatttg 1560

tgctacgcaa acactataaa ctggaaaaaa ctcttcggga cgcccaatca aaagaccaaa 1620

atcatgaaca acagagctga aaaggactgc aaggccacga accacgtctg taatccttta 1680

tgctcctcgg aaggctgctg gggccctgag cccacggact gtgtctcctg ccagaatgtg 1740

agcagaggca gggagtgcgt ggacaagtgc aacatcctgg agggggaacc gagggagttt 1800

gtggaaaatt ctgaatgcat ccagtgccat ccagaatgtc tgccccagac catgaacatc 1860

acctgtacag gccgggggcc agacaactgc atcaagtgtg cccactatgt tgatggtccc 1920

cactgtgtca agacctgccc ttcgggcatc atgggggaga acaacaccct ggtctggaag 1980

tttgcagatg ccaataacgt ctgccacctc tgccatgcaa actgtaccta tggatgtgct 2040

gggccaggcc ttaaaggatg tcaacaacca gaagggccaa agatcccatc catcgccact 2100

gggattgtgg gtggcctcct cttcatagta gtggtggccc ttgggatcgg cctcttcatg 2160

cgtcgacgtc agcttgtccg aaaacgtaca ctacgccgcc tgcttcaaga gagagagctc 2220

gtggaacctc tcacacccag cggagaagct ccgaaccaag cccacttgag gatattaaag 2280

gaaacagaat tcaaaaagat caaagttctg ggttcaggag catttggcac agtgtataag 2340

ggtctctgga tcccagaagg cgagaaagtg aaaatccctg tggccatcaa ggagttaaga 2400

gaagccacat ctcccaaagc caacaaggaa atccttgatg aagcctacgt gatggccagt 2460

gtggacaacc ctcatgtatg ccgcctcctg ggcatctgtc tgacctccac tgtccagctc 2520

attacacaac tcatgcccta tggttgcctc ctggactatg tccgagaaca taaggacaac 2580

attggctccc agtacctact caactggtgt gtgcagattg caaagggcat gaactacctg 2640

gaagaccggc gtttggtaca ccgtgacttg gcagccagga atgtactggt aaagacacca 2700

cagcatgtca agatcacaga ttttggactg gccaaactgc ttggtgctga ggagaaagaa 2760

taccatgcag aggggggcaa agtgcctatc aagtggatgg ctttggaatc aattttacac 2820

cgaatttata cacaccaaag cgacgtctgg agctatggag tcaccgtgtg ggaactgatg 2880

acctttgggt ccaagcctta tgatgggatc cctgcaagtg agatctcatc catcctagag 2940

aaaggagagc gccttccaca gccacctatc tgcaccatcg acgtctacat gatcatggtc 3000

aagtgctgga tgatagatgc tgatagccgc ccaaagttcc gagagttgat tctcgaattc 3060

tccaaaatgg ccagagaccc acagcgctac cttgttatcc agggggatga aaggatgcat 3120

ttgccgagcc ctacagactc caacttttac cgagccctga tggaggagga ggacatggaa 3180

gacgtagttg atgctgatga atacctcatc ccacagcaag gcttcttcaa cagcccatcc 3240

acgtcacgga ctccactctt gagctctctg agtgcaaata gcaacagttc cactgtggct 3300

tgcattaata gaaatgggag ctgccgtgtc aaagaagacg ccttcttgca acggtatagc 3360

tccgatccca ccagcgtcct gacagaggac aacatagatg acacattcct tcccgtgcct 3420

gaatatataa accaatctgt tcccaagagg ccggctggct ctgtgcagaa cccagtctat 3480

cacaatcagc ccctgcatcc agctcctgga agagacctgc attatcaaaa tccccatagc 3540

aatgcggtga gcaaccctga gtatctcaac actgcccagc cgacctgcct cagtagtggg 3600

tttgacagct ctgccctctg gatccagaaa ggcagccacc aaatgagcct ggacaaccct 3660

gactaccagc aggacttctt tcccaaagaa gccaagccga atggcatctt taagggcccc 3720

acagctgaaa atgcagagta cctgcgggtg gcaccgccaa gcagtgagtt tagtggagca 3780

tgacattgaa gaggcattgt accagttaca aaccggactt tccagaggcc caggaccaag 3840

ccatggcagc acctctgctc ctgacagcca tgtccacatt gtgtcaaatg tcaaaccctc 3900

agactggctt taaagcataa ctctgacggg ctttgtcact gagccaagaa gtgggcccct 3960

cccctgatgc tctttgggaa gttgaaggta tatctattgg tcttcgaact gtgaagattc 4020

cactgaaagg tatccatcga gaacattgtc cttttggaac agaaggttgc ctcatggtga 4080

ggtacatagg gggaaaaaaa acagacctat ggcgcttgct gacataggga actctgggat 4140

tcttgtcttt attgatttgc t 4161

<210> 13
<211> 4788
<212> DNA
<213> Canis familiaris

<400> 13
atgctttcac cacccaagtg tgagcaagtg caaggccgta gaggcaccgc aggaccggga 60

aaccgggaag agcagcggga tggggttgtc ctcctggggg gtgtcgctgt gagcggaggc 120

cacacacgtg gaattcccga gagcgccgag gaggagggag acctggagca gcgtccccgg 180

gtgggaaaag caggggtgag aaaagcagtg ggaggacgca gtcattgtct ccactgtctg 240

cagggctgcg aggtcgagga gaagcaggtc gcatctctgt tcacccggag gaagtcatgg 300

ggggacaagc ttggggcccc taaagcacag ggtcatggga accctgatcc tatacttcca 360

gacctcctgt taggccttcc tctgaagcag agctcggtga agtacagccc gtggttcaaa 420

tccagcgact gcctgttttt taagccaagt tttcgggcac gagccggtgc caggcccatt 480

cgtttggttc ttgtctgtgg ctgctttttg cacgctggcg gcggagcgga ggggctggga 540
cggacccttg gagaatgttc tgagcagcaa gctgggagtt ctcctgcggg aattctgaaa 600
ggcccatccg aggtgtggcc ccctcagctt tcagtgcccg gcttgccccc cccacccccg 660
ctcaaatcca cccttgcccc ccgaggctcc ccgtggaagg gctctgatat ctgcctgcac 720
ctcattgcca ggcttgcctg cgctcctgca acaggcacct ttggtcttgg ggcagctgag 780
gctcgtaggc tagctgaggg gctggacaag ccttacctgg caggctgtaa aacagttctg 840
gaaggtcagg attattttca gcatgcctac gacttgagga aagcgtgccg gggagagctt 900
agagaagtca ttcggggtca cgtcgtggct tattgtcgca accctgcacc tacatcagtg 960
gtggcatcgt tagggacctt gggattgggc aacgagtctg gccccatgtg catgcgttgc 1020
cgccagctgt gtacagatgt tccaagtgaa gggtgcaatc agagcagcct gtgcttccga 1080
gtgcgagcag cccagagctg tgaggccaca gaagagggaa atccccatag agttcaagag 1140
gatgtggcga ttgcttcata tttaatgtca gtgtcagtgc atggggttga tgatgtggtg 1200
tctgaacatg tgctcgctgc cgtggcacca aaacgcaagg gcagacggtc agtctgccaa 1260
ggcacgagta accggctcac ccagttgggc acttttgaag accactttct gagccttcag 1320
aggatgttca ataactgtga ggtggtcctg gggaatttgg aaattaccta catgcaaagg 1380
aattatgacc tttccttctt aaagaccatc caggaagttg ccgggtatgt attgattgcc 1440
ctcaacacgg tggagaagat tcctttggaa aacctgcaga tcatccgagg aaatgtgctc 1500
tatgaaaaca cccatgcctt atccgtcctc tccaactatg gctcaaataa aactggactg 1560
caggagcttc ccctgagaaa cttgcatgaa atactacaag gcgctgtgcg cttcagcaat 1620
aaccctgtcc tctgcaacgt ggaaaccatc cagtggcggg acatcgttga caacgatttt 1680
ataagcaaca tgtcgatgga catccagaac caagcgggca ggtgccaaaa gtgtgatcca 1740
agctgtccca atggaagttg ctggggtccg ggaaaggaga attgccagaa actgaccaaa 1800
atcatctgtg cccagcagtg ttcggggcgc tgccgcggca ggtcccccag cgactgctgc 1860
cacaaccagt gtgccgccgg ctgcacgggg ccccgcgaga gcgactgcct ggtctgccgc 1920
aagttccggg atgaagccac gtgcaaggac acctgcccgc ccctcatgct ctacaacccc 1980
accacctacc aaatggacgt caacccagag ggaaaataca gctttggtgc cacctgcgtg 2040
aagaaatgcc cccgcaacta cgtggtgaca gaccacggtt catgtgtccg cgcctgcagc 2100
tctgacagct acgaggtgga ggaggatggt gtccgcaagt gtaagaagtg tgagggccct 2160
tgtcgcaaag tttgtaatgg aatagggatt ggagagttca aagacacact ttccataaat 2220
gctacaaaca ttaaacactt caaaaactgc acgtcgatca gtggagacct tcatatcttg 2280

ccagtcgcat ttagaggtga ctccttcacg cataccctac ctctagatcc gaaggagctg 2340

gatatcctga aaactgtcaa ggaaataaca gggtttttgc tgattcaggc ctggcccgaa 2400

aataggactg acctccatgc tttcgagaac ctagaaatca tacgtggaag aacaaagcaa 2460

catggccagt tttctctggc ggtcgtcggc ctgaacataa cgtccttggg attacgctcc 2520

ctcaaggaga taagcgatgg agatgtgatc atctctggaa accgaaaatt gtgctatgca 2580

aatacaataa actggaagaa actctttggg acttcaagtc agaaaaccaa aattataaac 2640

aacaaagatg aaaaagcctg caaggccata ggccacgtct gccatccatt gtgctcctca 2700

gaaggctgct ggggcccagg gccgagagac tgcgtgtcct gccgaaacgt cagccgtggc 2760

aaggaatgtg tggaaaagtg caacattctg gagggggagc caagggagtt tgtggagaat 2820

tcggagtgca tacagtgcca tccggaatgc ctgccccagg ccatgaacat aacctgcaca 2880

ggacgggggc cggacagctg catcaagtgc gcccactaca tcgatggccc tcactgcgtc 2940

aagacctgcc cggctggcat catgggagaa aacaacaccc tggtctggaa gttttcggat 3000

ggcagccgca tgtgccacct gtgccatcca aactgcacct atggctgtga gggaccaggt 3060

cttgaaggct gtgcaaaacc tgggcccaag atcccatcca ttgctaccgg gattgtcggc 3120

ggcctcctct tggtggtggt ggtggccctt ggagtgggcc tctttttgcg ccggcgccac 3180

attgtccgga agcgcacgct tcgcagactg ctgcaagaaa gagagcttgt cgagcctctt 3240

acacccagcg gagaagctcc caaccaggct ctcttgagga tcttaaagga gacggagttc 3300

aaaaagatca aggtgctggg ctctggagca ttcggcacag tgtacaaggg actctggatc 3360

ccagaaggcg agaaggttaa aattcccgtg gccatcaagg aattgagaga agccacgtct 3420

cccaaagcca acaaggaaat tcttgatgaa gcctacgtga tggccagtgt ggacaatccc 3480

cacgtgtgcc gcctcctggg catctgcctg acgtccacgg tgcagctcat cacgcagctc 3540

atgccctttg gctgcctcct ggactatgtc cgcgagcaca aggacaacat cggctcccag 3600

cacctgctca actggtgtgt gcagattgca aagggcatga actacctgga agaccggcgc 3660

ttggtgcacc gcgacctggc ggccaggaac gtcctggtga agaccccgca gcacgtcaag 3720

atcacagatt ttgggctggc caaactgctg ggtgccgagg agaaagagta ccacgcggaa 3780

ggaggcaaag tgcctatcaa gtggatggct ttagaatcga ttttacaccg aatttatacc 3840

caccaaagcg atgtgtggag ctacggcgtc accgtgtggg agctgatgac cttcgggtcc 3900

aagccttacg acggtatccc tgcaagtgag atctccacca tcctggagaa gggagagcgc 3960

ctcccgcagc cgcccatatg caccatcgat gtctacatga tcatggtcaa gtgctggatg 4020

atagatgcag acagtcgccc aaaattccgt gagttgatca tcgaattctc caaaatggcc 4080

cgagacccgc agcgctacct tgtcatccag ggagatgaga ggatgcattt gccaagccct 4140

acagactcca atttttaccg cgccctgatg gacgaggagg acatggagga tgttgtggat 4200

gctgacgagt acctcatccc ccagcagggc ttcttccaca gcccctccac ttcccggacc 4260

cccctcctaa gttctctgag cgccaccagc aacagttcca acgtggcttg catcgaccga 4320

aatgggacct gtcccctcaa agaagacagc ttcttgcagc ggtacagctc agaccccact 4380

ggcaccttga cggaggacaa catagatgac actttcctcc cagcacccga atacataaac 4440

cagtctgttc ccaaaaggcc tgcgggttct gtccagaatc ccgtctatca caatcagcct 4500

ctaaatccag ctcctgccag agaccctcac taccaaaatc cccacagcaa cgcagtggac 4560

aaccctgagt atctcaacac ccaccccacc tgcgtcaaca gtgtcctcga caggcccagc 4620

ctctggaccc aggaggccaa ccaccaaatc agcctggaca accctgacta ccagcaggac 4680

ttctttccca aggaagccaa gtccaatggc atttttaagg gccctgcagc tgaaaatgca 4740

gactacctga gggtagcgcc accaagcagt gagtttattg gagcgtga 4788